(19) Europäisches Patentamt
European Patent Office
Office européen des brevets

(11) EP 1 797 898 A1

(12) EUROPEAN PATENT APPLICATION
published in accordance with Art. 158(3) EPC

(43) Date of publication:
**20.06.2007 Bulletin 2007/25**

(21) Application number: **05778503.2**

(22) Date of filing: **06.09.2005**

(51) Int Cl.:
***A61K 45/00*** *(2006.01)* ***A61K 38/57*** *(2006.01)*
***A61K 31/7088*** *(2006.01)* ***A61K 39/395*** *(2006.01)*
***A61K 45/06*** *(2006.01)* ***A61K 48/00*** *(2006.01)*
***A61P 3/06*** *(2006.01)* ***A61P 3/10*** *(2006.01)*
***A61P 9/10*** *(2006.01)* ***A61P 9/12*** *(2006.01)*
***A61P 13/12*** *(2006.01)* ***A61P 43/00*** *(2006.01)*
***C12N 15/09*** *(2006.01)* ***C12Q 1/527*** *(2006.01)*
***C12Q 1/68*** *(2006.01)* ***G01N 33/15*** *(2006.01)*
***G01N 33/50*** *(2006.01)* *C12N 9/88* *(2006.01)*

(86) International application number:
**PCT/JP2005/016694**

(87) International publication number:
**WO 2006/028241 (16.03.2006 Gazette 2006/11)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **08.09.2004 JP 2004261600**

(71) Applicant: **Takeda Pharmaceutical Company Limited**
**Osaka-shi,**
**Osaka 541-0045 (JP)**

(72) Inventors:
- **FUSE, Hiromitsu,**
**TAKEDA PHARMACEUTICAL COMP. LTD.**
**Osaka-shi,**
**Osaka532-8686 (JP)**
- **SHIBATA, Sachio,**
**TAKEDA PHARMACEUTICAL COMP. LTD.**
**Tsukuba-shi, Ibaraki,**
**300-4293 (JP)**
- **TOJO, Hideaki,**
**TAKEDA PHARMACEUTICAL COMP. LTD.**
**Tsukuba-shi, Ibaraki,**
**300-4293 (JP)**

(74) Representative: **Helbing, Jörg et al**
**Patentanwälte**
**von Kreisler-Selting-Werner,**
**Postfach 10 22 41**
**50462 Köln (DE)**

# (54) PREVENTIVE/THERAPEUTIC DRUG FOR ARTERIOSCLEROSIS

(57) The present invention provides preventive/therapeutic agents for arteriosclerosis, diabetes mellitus, etc., which comprise glucosylceramide or (and) lactosylceramide synthase inhibitors, expression inhibitors for glucosylceramide or (and) lactosylceramide synthase genes, methods and kits for screening the preventive/therapeutic agents, and so on.



Printed by Jouve, 75001 PARIS (FR)

SEQUENCE LISTING

<110> Takeda Pharmaceutical Company Limited

<120> Preventive/therapeutic drug for arteriosclerosis

<130> G07-0001

<140> PCT/JP2005/016694
<141> 2005-09-06

<150> JP 2004-261600
<151> 2004-09-08

<160> 12

<210> 1
<211> 394
<212> PRT
<213> Homo sapiens

<400> 1

```
Met Ala Leu Leu Asp Leu Ala Leu Glu Gly Met Ala Val Phe Gly Phe
                 5                  10                  15
Val Leu Phe Leu Val Leu Trp Leu Met His Phe Met Ala Ile Ile Tyr
             20                  25                  30
Thr Arg Leu His Leu Asn Lys Lys Ala Thr Asp Lys Gln Pro Tyr Ser
```

35 40 45

Lys Leu Pro Gly Val Ser Leu Leu Lys Pro Leu Lys Gly Val Asp Pro

50 55 60

Asn Leu Ile Asn Asn Leu Glu Thr Phe Phe Glu Leu Asp Tyr Pro Lys

65 70 75 80

Tyr Glu Val Leu Leu Cys Val Gln Asp His Asp Asp Pro Ala Ile Asp

85 90 95

Val Cys Lys Lys Leu Leu Gly Lys Tyr Pro Asn Val Asp Ala Arg Leu

100 105 110

Phe Ile Gly Gly Lys Lys Val Gly Ile Asn Pro Lys Ile Asn Asn Leu

115 120 125

Met Pro Gly Tyr Glu Val Ala Lys Tyr Asp Leu Ile Trp Ile Cys Asp

130 135 140

Ser Gly Ile Arg Val Ile Pro Asp Thr Leu Thr Asp Met Val Asn Gln

145 150 155 160

Met Thr Glu Lys Val Gly Leu Val His Gly Leu Pro Tyr Val Ala Asp

165 170 175

Arg Gln Gly Phe Ala Ala Thr Leu Glu Gln Val Tyr Phe Gly Thr Ser

180 185 190

His Pro Arg Tyr Tyr Ile Ser Ala Asn Val Thr Gly Phe Lys Cys Val

195 200 205

Thr Gly Met Ser Cys Leu Met Arg Lys Asp Val Leu Asp Gln Ala Gly

210 215 220

Gly Leu Ile Ala Phe Ala Gln Tyr Ile Ala Glu Asp Tyr Phe Met Ala

225 230 235 240

Lys Ala Ile Ala Asp Arg Gly Trp Arg Phe Ala Met Ser Thr Gln Val

245 250 255

Ala Met Gln Asn Ser Gly Ser Tyr Ser Ile Ser Gln Phe Gln Ser Arg
260 265 270

Met Ile Arg Trp Thr Lys Leu Arg Ile Asn Met Leu Pro Ala Thr Ile
275 280 285

Ile Cys Glu Pro Ile Ser Glu Cys Phe Val Ala Ser Leu Ile Ile Gly
290 295 300

Trp Ala Ala His His Val Phe Arg Trp Asp Ile Met Val Phe Phe Met
305 310 315 320

Cys His Cys Leu Ala Trp Phe Ile Phe Asp Tyr Ile Gln Leu Arg Gly
325 330 335

Val Gln Gly Gly Thr Leu Cys Phe Ser Lys Leu Asp Tyr Ala Val Ala
340 345 350

Trp Phe Ile Arg Glu Ser Met Thr Ile Tyr Ile Phe Leu Ser Ala Leu
355 360 365

Trp Asp Pro Thr Ile Ser Trp Arg Thr Gly Arg Tyr Arg Leu Arg Cys
370 375 380

Gly Gly Thr Ala Glu Glu Ile Leu Asp Val
385 390

<210> 2

<211> 1182

<212> DNA

<213> Homo sapiens

<400> 2

atggcgctgc tggacctggc cttggaggga atggccgtct tcgggttcgt cctcttcttg 60

gtgctgtggc tgatgcattt catggctatc atctacaccc gattacacct caacaagaag 120

gcaactgaca aacagcctta tagcaagctc ccaggtgtct ctcttctgaa accactgaaa 180

ggggtagatc ctaacttaat caacaacctg gaaacattct ttgaattgga ttatcccaaa 240

tatgaagtgc tcctttgtgt acaagatcat gatgatccag ccattgatgt atgtaagaag 300

cttcttggaa aatatccaaa tgttgatgct agattgttta taggtggtaa aaaagttggc 360

attaatccta aaattaataa tttaatgcca ggatatgaag ttgcaaagta tgatcttata 420

tggatttgtg atagtggaat aagagtaatt ccagatacgc ttactgacat ggtgaatcaa 480

atgacagaaa aagtaggctt ggttcacggg ctgccttacg tagcagacag acagggcttt 540

gctgccacct tagagcaggt atattttgga acttcacatc caagatacta tatctctgcc 600

aatgtaactg gtttcaaatg tgtgacagga atgtcttgtt taatgagaaa agatgtgttg 660

gatcaagcag gaggacttat agcttttgct cagtacattg ccgaagatta ctttatggcc 720

aaagcgatag ctgaccgagg ttggaggttt gcaatgtcca ctcaagttgc aatgcaaaac 780

tctggctcat attcaatttc tcagtttcaa tccagaatga tcaggtggac caaactacga 840

attaacatgc ttcctgctac aataatttgt gagccaattt cagaatgctt tgttgccagt 900

ttaattattg gatgggcagc ccaccatgtg ttcagatggg atattatggt atttttcatg 960

tgtcattgcc tggcatggtt tatatttgac tacattcaac tcaggggtgt ccagggtggc 1020

acactgtgtt tttcaaaact tgattatgca gtcgcctggt tcatccgcga atccatgaca 1080

atatacattt ttttgtctgc attatgggac ccaactataa gctggagaac tggtcgctac 1140

agattacgct gtgggggtac agcagaggaa atcctagatg ta 1182

<210> 3

<211> 382

<212> PRT

<213> Homo sapiens

<400> 3

Met Ser Val Leu Arg Arg Met Met Arg Val Ser Asn Arg Ser Leu Leu

5 10 15

Ala Phe Ile Phe Phe Phe Ser Leu Ser Ser Ser Cys Leu Tyr Phe Ile
20 25 30

Tyr Val Ala Pro Gly Ile Ala Asn Thr Tyr Leu Phe Met Val Gln Ala
35 40 45

Arg Gly Ile Met Leu Arg Glu Asn Val Lys Thr Ile Gly His Met Ile
50 55 60

Arg Leu Tyr Thr Asn Lys Asn Ser Thr Leu Asn Gly Thr Asp Tyr Pro
65 70 75 80

Glu Gly Asn Asn Ser Ser Asp Tyr Leu Val Gln Thr Thr Thr Tyr Leu
85 90 95

Pro Glu Asn Phe Thr Tyr Ser Pro Tyr Leu Pro Cys Pro Glu Lys Leu
100 105 110

Pro Tyr Met Arg Gly Phe Leu Asn Val Asn Val Ser Glu Val Ser Phe
115 120 125

Asp Glu Ile His Gln Leu Phe Ser Lys Asp Leu Asp Ile Glu Pro Gly
130 135 140

Gly His Trp Arg Pro Lys Asp Cys Lys Pro Arg Trp Lys Val Ala Val
145 150 155 160

Leu Ile Pro Phe Arg Asn Arg His Glu His Leu Pro Ile Phe Phe Leu
165 170 175

His Leu Ile Pro Met Leu Gln Lys Gln Arg Leu Glu Phe Ala Phe Tyr
180 185 190

Val Val Glu Gln Thr Gly Thr Gln Pro Phe Asn Arg Ala Met Leu Phe
195 200 205

Asn Val Gly Phe Lys Glu Ala Met Lys Asp Ser Val Trp Asp Cys Val
210 215 220

Ile Phe His Asp Val Asp His Leu Pro Glu Asn Asp Arg Asn Tyr Tyr

225 230 235 240

Gly Cys Gly Glu Met Pro Arg His Phe Ala Ala Lys Leu Asp Lys Tyr

245 250 255

Met Tyr Ile Leu Pro Tyr Lys Glu Phe Phe Gly Gly Val Ser Gly Leu

260 265 270

Thr Val Glu Gln Phe Arg Lys Ile Asn Gly Phe Pro Asn Ala Phe Trp

275 280 285

Gly Trp Gly Gly Glu Asp Asp Asp Leu Trp Asn Arg Val His Tyr Ala

290 295 300

Gly Tyr Asn Val Thr Arg Pro Glu Gly Asp Leu Gly Lys Tyr Lys Ser

305 310 315 320

Ile Pro His His His Arg Gly Glu Val Gln Phe Leu Gly Arg Tyr Lys

325 330 335

Leu Leu Arg Tyr Ser Lys Glu Arg Gln Tyr Ile Asp Gly Leu Asn Asn

340 345 350

Leu Ile Tyr Arg Pro Lys Ile Leu Val Asp Arg Leu Tyr Thr Asn Ile

355 360 365

Ser Val Asn Leu Met Pro Glu Leu Ala Pro Ile Glu Asp Tyr

370 375 380

<210> 4

<211> 1146

<212> DNA

<213> Homo sapiens

<400> 4

atgtctgtgc tcaggcggat gatgcgggtt tccaatcgct ctctcctcgc cttcatcttc 60

tttttctccc tctcttcgtc ctgtctgtac ttcatctatg tggccccagg catcgccaac 120
acatatctct ttatggtaca agctcgaggt ataatgttga gagaaaatgt gaaaacaata 180
ggtcatatga tcaggctgta cacaaataaa aacagtacgc tcaacggtac agattatccc 240
gaaggcaata attcaagtga ttatcttgtt caaacaacaa cgtatctccc ggaaaacttc 300
acatactcac catacctccc ctgtccagaa aagctgcctt atatgcgagg attcctcaat 360
gtcaatgtaa gcgaagtcag ttttgatgaa attcatcaac tcttctccaa ggatttagat 420
attgagccag gggtcattg gaggccaaaa gactgtaaac ccagatggaa ggtggcagtt 480
ctcattcctt tccgtaatcg ccatgaacat cttccaattt ttttcttaca tctgattcca 540
atgctccaga agcagcggct ggaatttgcg ttttatgtcg ttgaacagac tggcacacaa 600
ccttttaacc gtgcgatgct tttcaatgtg ggcttcaaag aggccatgaa agacagtgtc 660
tgggactgtg taatcttcca cgatgtggat catctacctg aaaatgaccg gaactattac 720
ggatgtggag aaatgccacg tcattttgct gcaaagctgg ataaatacat gtatattctt 780
ccatataaag aatttttgg tggtgtaagt gggctgacag tggaacaatt tagaaagatc 840
aatggttttc ctaatgcctt ctggggatgg ggaggagaag atgatgacct ttggaacaga 900
gttcactatg ctggatataa tgtaaccaga ccagagggag acttaggaaa atacaagtca 960
attcctcatc accatagagg tgaagtccag tttttaggac ggtataaatt actaaggtat 1020
tccaaggagc gtcagtacat cgatggactg aacaatttaa tatataggcc aaaaatactg 1080
gttgataggt tgtatacaaa catatctgta aacctcatgc cagagttagc tccaatcgaa 1140
gactat 1146

<210> 5
<211> 24
<212> DNA
<213> Artificial Sequence

<220>
<223> Primer

<400> 5
atttaatgcc aggatatgaa gttg 24

<210> 6
<211> 24
<212> DNA
<213> Artificial Sequence

<220>
<223> Primer

<400> 6
aattgaatat gagccagagt tttg 24

<210> 7
<211> 23
<212> DNA
<213> Artificial Sequence

<220>
<223> Primer

<400> 7
cgccgcttct accccaccgt cac 23

<210> 8

<211> 22

<212> DNA

<213> Artificial Sequence

<220>

<223> Primer

<400> 8

cgcgccagga agaagtttac aa 22

<210> 9

<211> 35

<212> DNA

<213> Artificial Sequence

<220>

<223> Primer

<400> 9

atttgcggcc gccaccatgg cgctgctgga cctgg 35

<210> 10

<211> 25

<212> DNA

<213> Artificial Sequence

<220>

<223> Primer

<400> 10

cgggatcctt atacatctag gattt 25

<210> 11

<211> 362

<212> PRT

<213> Homo sapiens

<400> 11

Met Arg Arg Pro Ser Leu Leu Leu Lys Asp Ile Leu Lys Cys Thr Leu
5 10 15

Leu Val Phe Gly Val Trp Ile Leu Tyr Ile Leu Lys Leu Asn Tyr Thr
20 25 30

Thr Glu Glu Cys Asp Met Lys Lys Met His Tyr Val Asp Pro Asp His
35 40 45

Val Lys Arg Ala Gln Lys Tyr Ala Gln Gln Val Leu Gln Lys Glu Cys
50 55 60

Arg Pro Lys Phe Ala Lys Thr Ser Met Ala Leu Leu Phe Glu His Arg
65 70 75 80

Tyr Ser Val Asp Leu Leu Pro Phe Val Gln Lys Ala Pro Lys Asp Ser
85 90 95

Glu Ala Glu Ser Lys Tyr Asp Pro Pro Phe Gly Phe Arg Lys Phe Ser
100 105 110

Ser Lys Val Gln Thr Leu Leu Glu Leu Leu Pro Glu His Asp Leu Pro
115 120 125

Glu His Leu Lys Ala Lys Thr Cys Arg Arg Cys Val Val Ile Gly Ser
130 135 140

Gly Gly Ile Leu His Gly Leu Glu Leu Gly His Thr Leu Asn Gln Phe
145 150 155 160

Asp Val Val Ile Arg Leu Asn Ser Ala Pro Val Glu Gly Tyr Ser Glu
165 170 175

His Val Gly Asn Lys Thr Thr Ile Arg Met Thr Tyr Pro Glu Gly Ala
180 185 190

Pro Leu Ser Asp Leu Glu Tyr Tyr Ser Asn Asp Leu Phe Val Ala Val
195 200 205

Leu Phe Lys Ser Val Asp Phe Asn Trp Leu Gln Ala Met Val Lys Lys
210 215 220

Glu Thr Leu Pro Phe Trp Val Arg Leu Phe Phe Trp Lys Gln Val Ala
225 230 235 240

Glu Lys Ile Pro Leu Gln Pro Lys His Phe Arg Ile Leu Asn Pro Val
245 250 255

Ile Ile Lys Glu Thr Ala Phe Asp Ile Leu Gln Tyr Ser Glu Pro Gln
260 265 270

Ser Arg Phe Trp Gly Arg Asp Lys Asn Val Pro Thr Ile Gly Val Ile
275 280 285

Ala Val Val Leu Ala Thr His Leu Cys Asp Glu Val Ser Leu Ala Gly
290 295 300

Phe Gly Tyr Asp Leu Asn Gln Pro Arg Thr Pro Leu His Tyr Phe Asp
305 310 315 320

Ser Gln Cys Met Ala Ala Met Asn Phe Gln Thr Met His Asn Val Thr
325 330 335

Thr Glu Thr Lys Phe Leu Leu Lys Leu Val Lys Glu Gly Val Val Lys

340 345 350

Asp Leu Ser Gly Gly Ile Asp Arg Glu Phe

355 360

<210> 12

<211> 1086

<212> DNA

<213> Homo sapiens

<400> 12

atgagaaggc ccagcttgtt attaaaagac atcctcaaat gtacattgct tgtgtttgga 60

gtgtggatcc tttatatcct caagttaaat tatactactg aagaatgtga catgaaaaaa 120

atgcattatg tggaccctga ccatgtaaag agagctcaga aatatgctca gcaagtcttg 180

cagaaggaat gtcgtcccaa gtttgccaag acatcaatgg cgctgttatt tgagcacagg 240

tatagcgtgg acttactccc ttttgtgcag aaggccccca aagacagtga agctgagtcc 300

aagtacgatc ctccttttgg gttccggaag ttctccagta aagtccagac cctcttggaa 360

ctcttgccag agcacgacct ccctgaacac ttgaaagcca agacctgtcg gcgctgtgtg 420

gttattggaa gcggaggaat actgcacgga ttagaactgg gccacaccct gaaccagttc 480

gatgttgtga taaggttaaa cagtgcacca gttgagggat attcagaaca tgttggaaat 540

aaaactacta taaggatgac ttatccagag ggcgcaccac tgtctgacct tgaatattat 600

tccaatgact tatttgttgc tgttttattt aagagtgttg atttcaactg gcttcaagca 660

atggtaaaaa aggaaaccct gccattctgg gtacgactct tcttttggaa gcaggtggca 720

gaaaaaatcc cactgcagcc aaaacatttc aggattttga atccagttat catcaaagag 780

actgcctttg acatccttca gtactcagag cctcagtcaa ggttctgggg ccgagataag 840

aacgtcccca caatcggtgt cattgccgtt gtcttagcca cacatctgtg cgatgaagtc 900

agtttggcgg gttttggata tgacctcaat caacccagaa cacctttgca ctacttcgac 960

agtcaatgca tggctgctat gaactttcag accatgcata atgtgacaac ggaaaccaag 1020

```
ttcctcttaa agctggtcaa agagggagtg gtgaaagatc tcagtggagg cattgatcgt   1080
gaattt                                                              1086
```

## Description

### TECHNICAL FIELD

The present invention relates to medicaments for preventing/treating arteriosclerosis, screening of the medicaments, diagnostic agents for arteriosclerosis, diagnostic markers for arteriosclerosis, and the like.

### BACKGROUND ART

The presence of atherosclerosis is an important factor in the development of ischemic organ events such as ischemic heart disease, cerebrovascular disorders, etc., which is ranked as the most frequent cause of death. Pathomorphological characteristics of arteriosclerotic lesions are fatty streaks in which cells (foam cells) made up mainly of macrophages with subendothelial deposits of cholesterol esters are accumulated and in advanced stages, fibrous plaques where infiltration of smooth muscle cells, macrophages, T cells, etc., cell necrotic patterns and lipid accumulation are observed. The site of lipid accumulation displays structural vulnerability, where plaque rupture triggered by hemodynamic force occurs, tissue factors and blood coagulation cascade factors are reacted with one another to cause exponential growth of thrombi. It has become clear that plaque rupture in the coronary artery to cause thrombotic occlusion is intimately linked to the development of a so-called acute coronary syndrome such as acute myocardial infarction, unstable angina, sudden cardiac death, etc. (Fuster, V. et al., N. Engl. J. Med., 326, pp. 242-250, 1992).

Some large-scale epidemiological surveys (4S study, CARE study, etc.) have shown that the most important risk factor for acute coronary syndrome is a serum cholesterol level, inter alia, low density lipoprotein-cholesterol level (LDL-C), and evidence that serum cholesterol-lowering therapy with statins which are HMG-CoA reductase inhibitors, reduced the incidence of heart disease events proves the significance of LDL as a risk factor.

Cell biological studies revealed that macrophages facilitate intracellular uptake of the oxidized LDL produced through modification of LDL as the primary plasma lipid carrier in blood, by a receptor called the scavenger receptor family. This receptor receives no negative feedback by the intracellular cholesterol level and the macrophages take up an excess of the oxidized LDL via the receptor to transform into foam cells.

It is reported that patients with hypercholesterolemia suffer from hyper LDLemia and LDL is prone to be oxidized, etc. In these respects, the quantification system of oxidized LDL in blood using specific antibodies against the oxidized LDL has been developed in recent years; the blood level increases in patient population at high risk of heart disease and moreover, the number of the oxidized LDL-positive macrophages increases at lesions (Ehara, S. et al., Circulation, 103, pp. 1955-1960, 2001), strongly suggesting that formation of atherosclerotic lesions based on macrophage foaming by oxidized LDL may occur in vivo. The oxidized LDL not only transforms macrophages into foam cells but also exhibits arteriosclerosis-inducing actions on endothelial cells, smooth muscle cells and macrophages, and apoptosis induction is considered as one of these actions. The present inventors reported that enzymes involved in the ceramide metabolism system play an important role for apoptosis of foam cell macrophages and have a relation to generation of arteriosclerosis (WO 03/78624).

Glucosylceramides are precursors of glycosphingolipids contained in plasma membrane, and their expression or activity modulations are known to be involved in biochemical or pathological/physiological processes (development, differentiation, malignant change, etc.). Knockdown mice of enzyme for glucosylceramide synthesis (glucosylceramide synthase) died before embryonic 9.5 days, which shows that the glycosphingolipids are indispensable for generation and differentiation (Biochim. Biophys. Acta, 1525, 1-12, 2001).

Lactosylceramides are substances formed by adding galactose to glucosylceramides and are substances of the branching point for multiple metabolism series (gala-series, ganglio-series, asialo-series, lacto-series, neolacto-series, globo-series and isoglobo-series) in the glycosphingolipid metabolism (Wakaru-Jikken-Igaku Series: Glycobiology, 44-52, 2002). It is known that when lactosylceramide is added to the culture system, the proliferation activity is increased in human smooth muscle cells (Biochem. Biophys. Res. Commun., 181, 554-61, 1991) and the expression of cell adhesion molecules such as ICAM-1 is increased in human endothelial cells (J. Biol. Chem., 273, 34349, 1998).

It is also reported that D-PDMP (D-threo-1-phenyl-2-decanoylamino-3-morpholino-1-propanol), which is an inhibitor of glucosylceramide synthase or lactosylceramide synthase, suppresses the action of TNF-$\alpha$ that inhibits phosphorylation of IRS-1 in response to insulin stimulation ( J. Biol. Chem., 277, 3085-3092, 2002).

### DISCLOSURE OF THE INVENTION

It has been keenly desired to establish a useful diagnostic marker using as a target the molecule that specifically changes its expression in animal model of arteriosclerosis, to elucidate the kinetics of plasma protein in arteriosclerosis and further to provide medicaments exhibiting effects of preventing/treating arteriosclerosis through a different mechanism than the heretofore known regulation of risk factors for arteriosclerosis.

The present inventors have made extensive efforts to solve the foregoing problems and as a result, found that glucosylceramide synthase inhibitors and lactosylceramide synthase inhibitors promote a cholesterol efflux activity in macrophage cells, and lactosylceramide suppresses the cholesterol efflux activity in the cells. Based on these findings, the present inventors have considered that drugs, etc. for inhibiting the activity or gene expression of glucosylceramide synthase or lactosylceramide synthase are useful for the prevention and (or) arteriosclerosis and continued further investigations. As a result, the present invention has come to be accomplished.

That is, the present invention provides the following features, and so on.

[1] A medicament for preventing/treating arteriosclerosis, which comprises a glucosylceramide synthase inhibitor or (and) a lactosylceramide synthase inhibitor. [1a] A medicament for preventing/treating diabetes mellitus, which comprises a glucosylceramide synthase inhibitor or (and) a lactosylceramide synthase inhibitor.

[2] A medicament for preventing/treating arteriosclerosis, which comprises an expression inhibitor of glucosylceramide synthase gene or (and) an expression inhibitor of lactosylceramide synthase gene.

[2a] A medicament for preventing/treating diabetes mellitus, which comprises an expression inhibitor of glucosylceramide synthase gene or (and) an expression inhibitor of lactosylceramide synthase gene.

[2b] The medicament for preventing/treating arteriosclerosis according to [1] described above, which further comprises an expression inhibitor of glucosylceramide synthase gene or (and) an expression inhibitor of lactosylceramide synthase gene.

[2c] The medicament for preventing/treating diabetes mellitus according to [1a] described above, which further comprises an expression inhibitor of glucosylceramide synthase gene or (and) an expression inhibitor of lactosylceramide synthase gene.

[3] The medicament according to [1] or [2] described above, wherein the glucosylceramide synthase is a protein comprising the same or substantially the same amino acid sequence as the amino acid sequence represented by SEQ ID NO: 1, its partial peptide, or a salt thereof.

[4] The medicament according to [1] or [2] described above, wherein the glucosylceramide synthase is a protein consisting of the amino acid sequence represented by SEQ ID NO: 1, or a salt thereof.

[5] The medicament according to [1] or [2] described above, wherein the lactosylceramide synthase is a protein comprising the same or substantially the same amino acid sequence as the amino acid sequence represented by SEQ ID NO: 3, its partial peptide, or a salt thereof.

[6] The medicament according to [1] or [2] described above, wherein the lactosylceramide synthase is a protein consisting of the amino acid sequence represented by SEQ ID NO: 3, or a salt thereof.

[7] The medicament according to [1], [2] or [2b] described above or [19] or [19b] described below, which further comprises a GM3 synthase inhibitor.

[7a] The medicament according to [1a], [2a] or [2c] described above or [19a] or [19c] described below, which further comprises a GM3 synthase inhibitor.

[8] The medicament according to [1], [2] or [2b] described above or [19] or [19b] described below, which further comprises an expression inhibitor of GM3 synthase gene.

[8a] The medicament according to [1a], [2a] or [2c] described above or [19a] or [19c] described below, which further comprises an expression inhibitor of GM3 synthase gene.

[9] The medicament according to [7] or [8] described above, wherein the GM3 synthase is a protein comprising the same or substantially the same amino acid sequence as the amino acid sequence represented by SEQ ID NO: 11, its partial peptide, or a salt thereof.

[10] The medicament according to [7] or [8] described above, wherein the GM3 synthase is a protein consisting of the amino acid sequence represented by SEQ ID NO: 11, or a salt thereof.

[11] An antisense polynucleotide, which comprises the entire or part of base sequence complementary or substantially complementary to a base sequence of a polynucleotide encoding a protein comprising the same or substantially the same amino acid sequence represented by SEQ ID NO: 1 or SEQ ID NO: 3, or a partial peptide thereof.

[12] A medicament, which comprises an antisense polynucleotide comprising the entire or part of base sequence complementary or substantially complementary to a base sequence of a polynucleotide encoding a protein comprising the same or substantially the same amino acid sequence represented by SEQ ID NO: 1 or (and) SEQ ID NO: 3, or a partial peptide thereof.

[13] The medicament according to [12] described above, which is a medicament for preventing/treating arteriosclerosis.

[13a] The medicament according to [12] described above, which is a medicament for preventing/treating diabetes mellitus.

[14] The medicament according to [13] described above, which further comprises an antisense polynucleotide comprising the entire or part of base sequence complementary or substantially complementary to a base sequence of a polynucleotide encoding a protein comprising the same or substantially the same amino acid sequence as the amino acid sequence represented by SEQ ID NO: 11, or a partial peptide thereof.

[15] A siRNA or shRNA for a polynucleotide encoding a protein comprising the same or substantially the same amino acid sequence represented by SEQ ID NO: 1 or SEQ ID NO: 3, or a partial peptide thereof.

[16] A medicament, which comprises a siRNA or shRNA for a polynucleotide encoding a protein comprising the same or substantially the same amino acid sequence represented by SEQ ID NO: 1 or (and) SEQ ID NO: 3, or a partial peptide thereof.

[17] The medicament according to [16] described above, which is a medicament for preventing/treating arteriosclerosis.

[17a] The medicament according to [16] described above, which is a medicament for preventing/treating diabetes mellitus.

[18] The medicament according to [17] described above, which further comprises a siRNA or shRNA for a polynucleotide encoding a protein comprising the same or substantially the same amino acid sequence as the amino acid sequence represented by SEQ ID NO: 11, or a partial peptide thereof.

[19] A medicament for preventing/treating arteriosclerosis, which comprises an antibody to a glucosylceramide synthase or (and) an antibody to a lactosylceramide synthase.

[19a] A medicament for preventing/treating diabetes mellitus, which comprises an antibody to a glucosylceramide synthase or (and) an antibody to a lactosylceramide synthase.

[19b] The medicament for preventing/treating arteriosclerosis according to [1], [2] or [2b] described above, which further comprises an antibody to a glucosylceramide synthase or (and) an antibody to a lactosylceramide synthase.

[19c] The medicament for preventing/treating diabetes mellitus according to [1a], [2a] or [2c] described above, which further comprises an antibody to a glucosylceramide synthase or (and) an antibody to a lactosylceramide synthase.

[20] The medicament according to [1], [2], [2b], [19] or [19b] described above, which further comprises an antibody to a GM3 synthase.

[20a] The medicament according to [1a], [2a], [2c], [19a] or [19c] described above, which further comprises an antibody to a GM3 synthase.

[21] A diagnostic agent for arteriosclerosis, which comprises an antibody to a glucosylceramide synthase or (and) an antibody to a lactosylceramide synthase.

[21a] A diagnostic agent for diabetes mellitus, which comprises an antibody to a glucosylceramide synthase or (and) an antibody to a lactosylceramide synthase.

[22] The diagnostic agent according to [21] described above, which further comprises an antibody to a GM3 synthase.

[22a] The diagnostic agent according to [21a] described above, which further comprises an antibody to a GM3 synthase.

[23] A method for diagnosis of arteriosclerosis, which comprises using an antibody to a glucosylceramide synthase or (and) an antibody to a lactosylceramide synthase. [23a] A method for diagnosis of diabetes mellitus, which comprises using an antibody to a glucosylceramide synthase or (and) an antibody to a lactosylceramide synthase.

[24] The method for diagnosis according to [23] described above, which further comprises using an antibody to a GM3 synthase.

[24a] The method for diagnosis according to [23a] described above, which further comprises using an antibody to a GM3 synthase.

[25] A diagnostic agent for arteriosclerosis, which comprises a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

[25a] A diagnostic agent for diabetes mellitus, which comprises a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

[26] The diagnostic agent according to [25] described above, which further comprises a polynucleotide encoding a protein comprising the same or substantially the same amino acid sequence as the amino acid sequence represented by SEQ ID NO: 11, or a partial peptide thereof.

[26a] The diagnostic agent according to [25a] described above, which further comprises a polynucleotide encoding a protein comprising the same or substantially the same amino acid sequence as the amino acid sequence represented by SEQ ID NO: 11, or a partial peptide thereof.

[27] A method for diagnosis of arteriosclerosis, which comprises using a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

[27a] A method for diagnosis of diabetes mellitus, which comprises using a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

[28] The method for diagnosis according to [27] described above, which further comprises using a polynucleotide encoding a protein comprising the same or substantially the same amino acid sequence as the amino acid sequence represented by SEQ ID NO: 11, or a partial peptide thereof.

[28a] The method for diagnosis according to [27a] described above, which further comprises using a polynucleotide encoding a protein comprising the same or substantially the same amino acid sequence as the amino acid sequence represented by SEQ ID NO: 11, or a partial peptide thereof.

[29] Use of an antibody to a glucosylceramide synthase or (and) an antibody to a lactosylceramide synthase to manufacture a diagnostic agent for arteriosclerosis. [29a] The use according to [29] described above, which further comprises using an antibody to a GM3 synthase.

[29b] Use of an antibody to a glucosylceramide synthase or (and) an antibody to a lactosylceramide synthase to manufacture a diagnostic agent for diabetes mellitus. [29c] The use according to [29b] described above, which further comprises using an antibody to a GM3 synthase.

[30] Use of a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof, to manufacture a diagnostic agent for arteriosclerosis.

[30a] The use according to [30] described above, which further comprises using a polynucleotide encoding a protein comprising the same or substantially the same amino acid sequence represented by SEQ ID NO: 11, or a partial peptide thereof. [30b] Use of a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof, to manufacture a diagnostic agent for diabetes mellitus.

[30c] The use according to [30b] described above, which further comprises using a polynucleotide encoding a protein comprising the same or substantially the same amino acid sequence represented by SEQ ID NO: 11, or a partial peptide thereof.

[31] A method for diagnosis of arteriosclerosis, which comprises assaying the level of a glucosylceramide or (and) a lactosylceramide in plasma of a mammal.

[31a] A method for diagnosis of diabetes mellitus, which comprises assaying the level of a glucosylceramide or (and) a lactosylceramide in plasma of a mammal.

[32] The method for diagnosis according to [31] described above, which further comprises assaying the level of GM3.

[32a] The method for diagnosis according to [31a] described above, which further comprises assaying the level of GM3.

[33] Use of a glucosylceramide or (and) a lactosylceramide as a diagnostic marker for arteriosclerosis.

[33a] Use of a glucosylceramide or (and) a lactosylceramide as a diagnostic marker for diabetes mellitus.

[34] The use according to [33] described above, which further comprises use of GM3.

[34a] The use according to [33a] described above, which further comprises use of GM3.

[35] A method of screening an agent for preventing/treating arteriosclerosis, which comprises using a protein comprising the same or substantially the same amino acid sequence as the amino acid sequence represented by SEQ ID NO: 1, its partial peptide, or a salt thereof, or (and) a protein comprising the same or substantially the same amino acid sequence as the amino acid sequence represented by SEQ ID NO: 3, its partial peptide, or a salt thereof.

[35a] A method of screening an agent for preventing/treating diabetes mellitus, which comprises using a protein comprising the same or substantially the same amino acid sequence as the amino acid sequence represented by SEQ ID NO: 1, its partial peptide, or a salt thereof, or (and) a protein comprising the same or substantially the same amino acid sequence as the amino acid sequence represented by SEQ ID NO: 3, its partial peptide, or a salt thereof.

[36] The screening method according to [35] described above, which further comprises using a protein comprising the same or substantially the same amino acid sequence as the amino acid sequence represented by SEQ ID NO: 11, its partial peptide, or a salt thereof.

[36a] The screening method according to [35a] described above, which further comprises using a protein comprising the same or substantially the same amino acid sequence as the amino acid sequence represented by SEQ ID NO: 11, its partial peptide, or a salt thereof.

[37] A kit for screening an agent for preventing/treating arteriosclerosis, which comprises a protein comprising the same or substantially the same amino acid sequence as the amino acid sequence represented by SEQ ID NO: 1, its partial peptide, or a salt thereof or (and) a protein comprising the same or substantially the same amino acid sequence as the amino acid sequence represented by SEQ ID NO: 3, its partial peptide, or a salt thereof.

[37a] A kit for screening an agent for preventing/treating diabetes mellitus, which comprises a protein comprising the same or substantially the same amino acid sequence as the amino acid sequence represented by SEQ ID NO: 1, its partial peptide, or a salt thereof or (and) a protein comprising the same or substantially the same amino acid sequence as the amino acid sequence represented by SEQ ID NO: 3, its partial peptide, or a salt thereof.

[38] The screening kit according to [37] described above, which further comprises a protein comprising the same or substantially the same amino acid sequence as the amino acid sequence represented by SEQ ID NO: 11, its partial peptide, or a salt thereof.

[38a] The screening kit according to [37a] described above, which further comprises a protein comprising the same or substantially the same amino acid sequence as the amino acid sequence represented by SEQ ID NO: 11, its partial peptide, or a salt thereof.

[39] A method of screening an agent for preventing/treating arteriosclerosis, which comprises using a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

[39a] A method of screening an agent for preventing/treating diabetes mellitus, which comprises using a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

[40] A method of screening an agent for preventing/treating arteriosclerosis, which comprises using a polynucleotide encoding a protein comprising the same or substantially the same amino acid sequence as the amino acid sequence represented by SEQ ID NO: 11, or a partial peptide thereof.

[40a] A method of screening an agent for preventing/treating diabetes mellitus, which comprises using a polynucleotide encoding a protein comprising the same or substantially the same amino acid sequence as the amino acid sequence represented by SEQ ID NO: 11, or a partial peptide thereof.

[41] A kit for screening an agent for preventing/treating arteriosclerosis, which comprises a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

[41a] A kit for screening an agent for preventing/treating diabetes mellitus, which comprises a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

[42] The screening kit according to [41] described above, which further comprises a polynucleotide encoding a protein comprising the same or substantially the same amino acid sequence as the amino acid sequence represented by SEQ ID NO: 11, or a partial peptide thereof.

[42a] The screening kit according to [41a] described above, which further comprises a polynucleotide encoding a protein comprising the same or substantially the same amino acid sequence as the amino acid sequence represented by SEQ ID NO: 11, or a partial peptide thereof.

[43] A method of screening an agent for preventing/treating arteriosclerosis, which comprises assaying the activity or level of a protein comprising the same or substantially the same amino acid sequence represented by SEQ ID NO: 1, its partial peptide, or a salt thereof, or (and) a protein comprising the same or substantially the same amino acid sequence as the amino acid sequence represented by SEQ ID NO: 3, its partial peptide, or a salt thereof.

[43a] A method of screening an agent for preventing/treating diabetes mellitus, which comprises assaying the activity or level of a protein comprising the same or substantially the same amino acid sequence represented by SEQ ID NO: 1, its partial peptide, or a salt thereof, or (and) a protein comprising the same or substantially the same amino acid sequence as the amino acid sequence represented by SEQ ID NO: 3, its partial peptide, or a salt thereof.

[44] The screening method according to [43] described above, which further comprises assaying the activity or level of a protein comprising the same or substantially the same amino acid sequence represented by SEQ ID NO: 11, its partial peptide, or a salt thereof.

[44a] The screening method according to [43a] described above, which further comprises assaying the activity or level of a protein comprising the same or substantially the same amino acid sequence represented by SEQ ID NO: 11, its partial peptide, or a salt thereof.

[45] A method of screening an agent for preventing/treating arteriosclerosis, which comprises assaying the level of a polynucleotide encoding a protein comprising the same or substantially the same amino acid sequence as the amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

[45a] A method of screening an agent for preventing/treating diabetes mellitus, which comprises assaying the level of a polynucleotide encoding a protein comprising the same or substantially the same amino acid sequence as the amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

[46] The screening method according to [45] described above, which further comprises assaying the level of a polynucleotide encoding a protein comprising the same or substantially the same amino acid sequence as the amino acid sequence represented by SEQ ID NO: 11, or a partial peptide thereof.

[46a] The screening method according to [45a] described above, which further comprises assaying the level of a polynucleotide encoding a protein comprising the same or substantially the same amino acid sequence as the amino acid sequence represented by SEQ ID NO: 11, or a partial peptide thereof.

[47] A method of preventing/treating arteriosclerosis, which comprises inhibiting the activity of a glucosylceramide synthase or (and) a lactosylceramide synthase.

[47a] A method of preventing/treating diabetes mellitus, which comprises inhibiting the activity of a glucosylceramide synthase or (and) a lactosylceramide synthase.

[48] The method according to [47] described above or [49] or [49b] described below, which further comprises inhibiting the activity of a GM3 synthase.

[48a] The method according to [47a] described above or [49a] or [49c] described below, which further comprises inhibiting the activity of a GM3 synthase.

[49] A method of preventing/treating arteriosclerosis, which comprises inhibiting the expression of a glucosylceramide synthase gene or (and) a lactosylceramide synthase gene.

[49a] A method of preventing/treating diabetes mellitus, which comprises inhibiting the expression of a glucosylceramide synthase gene or (and) a lactosylceramide synthase gene.

[49b] The method of preventing/treating arteriosclerosis according to [47] described above, which further comprises inhibiting the expression of a glucosylceramide synthase gene or (and) a lactosylceramide synthase gene.

[49c] The method of preventing/treating diabetes mellitus according to [47a] described above, which further comprises inhibiting the expression of a glucosylceramide synthase gene or (and) a lactosylceramide synthase gene.

[50] The method according to [47], [49] or [49b] described above, which further comprises inhibiting the expression of a GM3 synthase gene.

[50a] The method according to [47a], [49a] or [49c] described above, which further comprises inhibiting the expression of a GM3 synthase gene.

[51] A method of preventing/treating arteriosclerosis, which comprises administering to a mammal an effective dose of a glucosylceramide synthase inhibitor or (and) a lactosylceramide synthase inhibitor.

[51a] A method of preventing/treating diabetes mellitus, which comprises administering to a mammal an effective dose of a glucosylceramide synthase inhibitor or (and) a lactosylceramide synthase inhibitor.

[52] The method according to [51] described above, or [53] or [53b] described below, which further comprises administering an effective dose of a GM3 synthase inhibitor.

[52a] The method according to [51a] described above, or [53a] or [53c] described below, which further comprises administering an effective dose of a GM3 synthase inhibitor.

[53] A method of preventing/treating arteriosclerosis, which comprises administering to a mammal an effective dose of an expression inhibitor of glucosylceramide synthase gene or (and) lactosylceramide synthase gene.

[53a] A method of preventing/treating diabetes mellitus, which comprises administering to a mammal an effective dose of an expression inhibitor of glucosylceramide synthase gene or (and) lactosylceramide synthase gene.

[53b] The method of preventing/treating arteriosclerosis according to [51] described above, which comprises administering to a mammal an effective dose of an expression inhibitor of glucosylceramide synthase gene or (and) lactosylceramide synthase gene.

[53c] The method of preventing/treating diabetes mellitus according to [51a] described above, which comprises administering to a mammal an effective dose of an expression inhibitor of glucosylceramide synthase gene or (and) lactosylceramide synthase gene.

[54] The method according to [51], [53] or [53b] described above, which further comprises administering an effective dose of an expression inhibitor of GM3 synthase gene.

[54a] The method according to [51a], [53a] or [53c] described above, which further comprises administering an effective dose of an expression inhibitor of GM3 synthase gene.

[55] Use of a glucosylceramide synthase inhibitor or (and) a lactosylceramide synthase inhibitor to manufacture an agent for preventing/treating arteriosclerosis. [55a] Use of a glucosylceramide synthase inhibitor or (and) a lactosylceramide synthase inhibitor to manufacture an agent for preventing/treating diabetes mellitus.

[56] The use according to [55] described above or [57] or [57b] described below, which further comprises use of a GM3 synthase inhibitor.

[56a] The use according to [55a] described above or [57a] or [57c] described below, which further comprises use of a GM3 synthase inhibitor.

[57] Use of an expression inhibitor of glucosylceramide synthase gene or (and) lactosylceramide synthase gene to manufacture an agent for preventing/treating arteriosclerosis.

[57a] Use of an expression inhibitor of glucosylceramide synthase gene or (and) lactosylceramide synthase gene to manufacture an agent for preventing/treating diabetes mellitus.

[57b] The use according to [55] described above, which further comprises use of an expression inhibitor of glucosylceramide synthase gene or (and) lactosylceramide synthase gene.

[57c] The use according to [55a] described above, which further comprises use of an expression inhibitor of glucosylceramide synthase gene or (and) lactosylceramide synthase gene.

[58] The use according to [55], [57] or [57b] described above, which further comprises use of an expression inhibitor of GM3 synthase gene.

[58a] The use according to [55a], [57a] or [57c] described above, which further comprises use of an expression inhibitor of GM3 synthase gene.

BEST MODE FOR CARRYING OUT THE INVENTION

The glucosylceramide synthase (EC_number=2.4.1.80) used in the present invention is also called UGCG (UDP-glucose: ceramide glucosyltransferase), ceramide: UDPGlc glucosyltransferase, uridine diphosphoglucose-ceramide glucosyltransferase, ceramide: UDP-glucose glucosyltransferase, ceramide glucosyltransferase, etc., and is an enzyme which catalyzes the synthesis reaction of glucosylceramide biosynthesized by transferring glucose from UDP-glucose onto the hydroxy group at the 1-position of ceramide.

The lactosylceramide synthase (EC_number=2.4.1.-) used in the present invention is also called B4GLT6, GalT-2, UDP-Gal:betaGlcNAc beta 1,4-galactosyltransferase polypeptide 6, etc., and is an enzyme which catalyzes the synthesis reaction of lactosylceramide biosynthesized by transferring galactose from UDP-galactose onto glucosylceramide by the β-1,4 bond.

The GM3 synthase (Ganglioside GM3 synthase; EC_number=2.4.99.9) is also called SIAT9 (sialyltransferese 9), SAT-1 (sialyltransferase I), CMP-sialic acid: lactosylceramide α-2,3-sialyltransferase (CMP-NeuAc: lactosylceramide alpha-2,3-sialyltransferase), etc., and is an enzyme which catalyzes the synthesis reaction of GM3 (α-N-acetylneuraminyl-2,3-β-D-galactosyl-1,4-β-D-glucosylceramide) biosynthesized by transferring sialic acid from CMP-sialic acid (CMP-N-acetylneuraminate) onto the non-reducing terminal galactose by the α-2,3 bond.

The glucosylceramide synthase used in the present invention includes, for example, the protein comprising the same or the substantially the same amino acid sequence represented by SEQ ID NO: 1 (hereinafter this protein is sometimes referred to as the protein A of the present invention or the protein A used in the present invention), the lactosylceramide synthase includes, for example, the protein comprising the same or the substantially the same amino acid sequence represented by SEQ ID NO: 3 (hereinafter this protein is sometimes referred to as the protein B of the present invention or the protein B used in the present invention), the GM3 synthase includes, for example, the protein comprising the same or the substantially the same amino acid sequence represented by SEQ ID NO: 11 (hereinafter this protein is sometimes referred to as the protein C of the present invention or the protein C used in the present invention) (hereinafter, these protein are sometimes collectively referred to as the protein of the present invention or the protein used in the present invention).

The protein used in the present invention may be any protein derived from any cell of human and warm-blooded animals (e.g., guinea pig, rat, mouse, fowl, rabbit, swine, sheep, bovine, monkey, etc.); any cell (e.g., hepatocyte, splenocyte, nerve cells, glial cells, β cells of pancreas, bone marrow cells, mesangial cells, Langerhans' cells, epidermic cells, epithelial cells, goblet cell, endothelial cells, smooth muscle cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testicle, ovary, placenta, uterus, bone, joint, skeletal muscle, etc.; the proteins may also be synthetic proteins.

The amino acid sequence comprising substantially the same amino acid sequence as that represented by SEQ ID NO: 1, SEQ ID NO: 3 or SEQ ID NO: 11 includes amino acid sequences having at least about 50% homology, preferably at least about 60% homology, more preferably at least about 70% homology, still more preferably at least about 80% homology, much more preferably at least about 90% homology and most preferably at least about 95% homology, to the amino acid sequence shown by SEQ ID NO: 1; etc.

Homology of the amino acid sequences can be measured under the following conditions (an expectation value =10; gaps are allowed; matrix = BLOSUM62; filtering = OFF) using a homology scoring algorithm NCBI BLAST (National Center for Biotechnology Information Basic Local Alignment Search Tool).

Preferred examples of the protein comprising substantially the same amino acid sequence as the amino acid sequence represented by SEQ ID NO: 1, SEQ ID NO: 3 or SEQ ID NO: 11 include proteins comprising substantially the same amino acid sequence as the amino acid sequence represented by SEQ ID NO: 1, SEQ ID NO: 3 or SEQ ID NO: 11 and having an activity substantially equivalent to that of the protein comprising the amino acid sequence represented by SEQ ID NO: 1, SEQ ID NO: 3 or SEQ ID NO: 11, and the like.

Examples of the protein comprising substantially the same amino acid sequence as the amino acid sequence represented by SEQ ID NO: 1 include proteins comprising the amino acid sequence represented by SEQ ID NO: 1, and the protein consisting of the amino acid sequence represented by SEQ ID NO: 1 is preferably used.

As the substantially equivalent activities, there are, for example, activities of enzymes which catalyze the synthesis reaction of glucosylceramide (glucosylceramide synthase activity), or the like. The substantially equivalent is used to mean that the nature of these properties is equivalent in terms of quality (e.g., physiologically or pharmacologically). Accordingly, the glucosylceramide synthase activity is preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

Examples of the protein comprising substantially the same amino acid sequence as the amino acid sequence represented by SEQ ID NO: 3 include proteins comprising the amino acid sequence represented by SEQ ID NO: 3 and, preferably used is the protein consisting of the amino acid sequence represented by SEQ ID NO:3.

As the substantially equivalent properties, there are, for example, activities of enzymes which catalyze the synthesis reaction of lactosylceramide (lactosylceramide synthase activity), and the like. The substantially equivalent is used to mean that the nature of these properties is equivalent in terms of quality (e.g., physiologically or pharmacologically). Thus, the lactosylceramide synthase activity is preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

Examples of the protein comprising substantially the same amino acid sequence as the amino acid sequence represented by SEQ ID NO: 11 include proteins comprising the amino acid sequence represented by SEQ ID NO: 11, and, preferably used is the protein consisting of the amino acid sequence represented by SEQ ID NO: 11.

As the substantially equivalent properties, there are, for example, activities of enzymes which catalyze the synthesis reaction of GM3 (GM3 synthase activity), and the like. The substantially equivalent is used to mean that the nature of these properties is equivalent in terms of quality (e.g., physiologically or pharmacologically). Thus, the GM3 synthase activity is preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

The glucosylceramide synthase activity is assayed by publicly known methods, e.g., the method described in Methods in ENZYMOLOGY, 311, 50-59, 2000, etc., or its modifications. Specifically, glucosylceramide synthase, ceramide and UDP-radioactive glucose are reacted and the radioactivity in the chloroform layer after chloroform-methanol extraction is assayed. Alternatively, glucosylceramide synthase, fluorescence-labeled ceramide and UDP-glucose are reacted, the chloroform layer after chloroform-methanol extraction is recovered and solidified to dryness, glucosylceramide is then separated by silica 60 thin-layer chromatography (thin-layer chromatography, TLC) and the fluorescence intensity is detected and assayed on a device enabling to perform fluorescent imaging (e.g., LAS-1000 (manufactured by Fuji Film Co., Ltd.) etc.).

The lactosylceramide synthase activity is assayed by publicly known methods, e.g., the method described in Methods in ENZYMOLOGY, 311, 73-81, 2000, etc., or its modifications. Specifically, lactosylceramide synthase, glucosylceramide and UDP-radioactive galactose are reacted and the radioactivity in the chloroform layer after chloroform-methanol extraction is assayed.

In addition, the level of glucosylceramide or the level of lactosylceramide contained in cells or tissues is determined by publicly known methods, e.g., the method described in Kiso-Seikagaku-Jikkenho, 5, Chapter 11, 135-141, 2000, or its modifications.

Specifically, 2 ml of chloroform-methanol (2:1) is added to cell pellets (3 mg to 5 mg of protein), which is ultrasonicated for about 5 minutes and then shaken in an incubator at 37°C for an hour, followed by extraction. Subsequently, the extract is centrifuged to divide into the pellets and the supernatant (lipid extract). The pellets are again shaken in 1 ml of chloroform-methanol-water (1:2:0.8) at 37°C for 2 hours for reextraction. After centrifugation, the supernatant was combined with the supernatant obtained by the first centrifugation and the mixture is used as total lipids. Furthermore, 20-fold volume of chloroform-methanol (2:1) is added to the tissue homogenate and the mixture is ultrasonicated for about 5 minutes, followed by shaking in an incubator at 37°C for an hour. After methanol is added to the extract to adjust the proportion of chloroform-methanol to 1:1, the mixture is centrifuged to divide into the pellets and the supernatant (lipid extract). The pellets are again shaken in 2 ml of chloroform-methanol-water (1:2:0.8) at 37°C for 2 hours for reextraction. After centrifugation, the supernatant was combined with the supernatant obtained by the first centrifugation and the mixture is used as total lipids. The total lipids are passed through an anion exchange column converted into acetate type and eluted by chloroform-methanol-water (30:60:8) in a volume of 4 to 5 times the column volume. The solvent is removed from the eluate using an evaporator and the residue is again dissolved in 0.5 ml of chloroform-methanol (1:1). After 0.025 ml of 4M NaOH is added to the solution, the mixture is warmed at 37°C for 2 hours and 0.025 ml of 2M acetic acid is added thereto. A sample is applied to gel filtration chromatography to elute with chloroform-methanol-water (5:5:1). The solvent is removed from the eluted fraction using an evaporator and the residue is dissolved in chloroform-methanol (1:1). The solution is used as a sample for thin-layer chromatography (TLC). The sample is spotted onto a HPTLC plate (Merck), which is then developed with chloroform-methanol-water (65:25:4), etc., and an anthrone reagent, orcinol-sulfuric reagent, etc. for giving the color of a hexose are used to detect glucosylceramide or lactosylceramide. Quantification is performed by densitometry using an internal standard of glucosylceramide or lactosylceramide with known concentrations.

The GM3 synthase activity is assayed by publicly known methods, e.g., a method which involves incubating CMP-sialic acid (10-100 nmol) and an enzyme for a given time at 37°C using lactosylceramide as substrate and quantitatively determining the GM3 produced by the enzymatic reaction (e.g., the method described in Methods in ENZYMOLOGY, 311, 82-94, 2000, etc.), or modifications thereof.

Specifically, GM3 on the cell surface in GM3 synthase expressed cell line (e.g., mouse melanoma B16-F1, etc.) or in the cells is quantitatively detected using an anti-GM3 antibody. The cell surface GM3 is fixed in formaldehyde, etc. and reacted with anti-GM3 antibody (e.g., Clone M2590, etc.), which is then reacted with HRP-labeled anti-mouse IgM antibody or AP-labeled anti-mouse IgM antibody, followed by detecting GM3 in the chemiluminescence system or in the color forming system (J. Biol. Chem., 277, 47028-47034, 2002). Alternatively, the cells fixed in formaldehyde, etc. or the cells in a non-fixed, suspended state are reacted with anti-GM3 antibody (e.g., Clone M2590, etc.), which is then reacted with FITC-labeled anti-mouse IgM antibody, etc., followed by analysis by using FACS (J. Biol. Chem., 277, 3085-3092, 2002). Also, the intracellular GM3 level becomes detectable by pretreating the cells in Triton X-100, etc. (J. Cell. Phys., 141, 573-583, 1989).

In the specification, the "inhibition of glucosylceramide synthase" can be either of the inhibition of glucosylceramide synthase activity or the inhibition of glucosylceramide synthase production (preferably the inhibition of glucosylceramide synthase activity).

In the specification, the "glucosylceramide synthase inhibitor" can be either of the compound or its salt that inhibits the activity of the protein comprising the same or substantially the same amino acid sequence as the amino acid sequence represented by SEQ ID NO: 1, its partial peptide, or a salt thereof; or the compound or its salt that inhibits the production of the protein comprising the same or substantially the same amino acid sequence as the amino acid sequence represented by SEQ ID NO: 1, its partial peptide, or a salt thereof (preferably, the compound or its salt that inhibits the activity of the protein comprising the same or substantially the same amino acid sequence as the amino acid sequence represented by SEQ ID NO: 1, its partial peptide, or a salt thereof).

In the specification, the "expression inhibitor of glucosylceramide synthase gene" used includes the compound or its salt that inhibits the expression of the polynucleotide encoding the protein comprising the same or substantially the same amino acid sequence as the amino acid sequence represented by SEQ ID NO: 1, or its partial peptide, etc.

In the specification, the "inhibition of lactosylceramide synthase" can be either of the inhibition of lactosylceramide synthase activity or the inhibition of lactosylceramide synthase production (preferably the inhibition of lactosylceramide synthase activity).

In the specification, the " lactosylceramide synthase inhibitor" can be either of the compound or its salt that inhibits the activity of the protein comprising the same or substantially the same amino acid sequence as the amino acid sequence represented by SEQ ID NO: 3, its partial peptide, or a salt thereof; or the compound or its salt that inhibits the production of the protein comprising the same or substantially the same amino acid sequence as the amino acid sequence represented by SEQ ID NO: 3, its partial peptide, or a salt thereof (preferably, the compound or its salt that inhibits the activity of the protein comprising the same or substantially the same amino acid sequence as the amino acid sequence represented by SEQ ID NO: 3, its partial peptide, or a salt thereof).

In the specification, the "expression inhibitor of lactosylceramide synthase gene" used includes the compound or its salt that inhibits the expression of the polynucleotide encoding the protein comprising the same or substantially the same amino acid sequence as the amino acid sequence represented by SEQ ID NO: 3, or its partial peptide, etc.

In the specification, the "inhibition of GM3 synthase" can be either of the inhibition of GM3 synthase activity or the inhibition of GM3 synthase production (preferably the inhibition of GM3 synthase activity).

In the specification, the "GM3 synthase inhibitor" can be either of the compound or its salt that inhibits the activity of the protein comprising the same or substantially the same amino acid sequence as the amino acid sequence represented by SEQ ID NO: 11, its partial peptide, or a salt thereof; or the compound or its salt that inhibits the production of the protein comprising the same or substantially the same amino acid sequence as the amino acid sequence represented by SEQ ID NO: 11, its partial peptide, or a salt thereof (preferably, the compound or its salt that inhibits the activity of the protein comprising the same or substantially the same amino acid sequence as the amino acid sequence represented by SEQ ID NO: 11, its partial peptide, or a salt thereof).

In the specification, the "expression inhibitor of GM3 synthase gene" used includes the compound or its salt that inhibits the expression of the polynucleotide encoding the protein comprising the same or substantially the same amino acid sequence as the amino acid sequence represented by SEQ ID NO: 11, or its partial peptide, etc.

Examples of the protein used in the present invention include so-called muteins such as proteins comprising (1) the amino acid sequence represented by SEQ ID NO: 1, SEQ ID NO: 3 or SEQ ID NO: 11, of which at least 1 or 2 (e.g., about 1 to about 100, preferably about 1 to about 30, more preferably about 1 to about 10 and most preferably several (1 to 5)) amino acids are deleted; (2) the amino acid sequence represented by SEQ ID NO: 1, SEQ ID NO: 3 or SEQ ID NO: 11, to which at least 1 or 2 (e.g., about 1 to about 100, preferably about 1 to about 30, more preferably about 1 to about 10 and most preferably several (1 to 5)) amino acids are added; (3) the amino acid sequence represented by SEQ ID NO: 1, SEQ ID NO: 3 or SEQ ID NO: 11, in which at least 1 or 2 (e.g., about 1 to about 100, preferably about 1 to about 30, more preferably about 1 to about 10 and most preferably several (1 to 5)) amino acids are inserted; (4) the amino acid sequence represented by SEQ ID NO: 1, SEQ ID NO: 3 or SEQ ID NO: 11, in which at least 1 or 2 (e.g., about 1 to about 100, preferably about 1 to about 30, more preferably about 1 to about 10 and most preferably several (1 to 5)) amino acids are substituted by other amino acids; or (5) a combination of these amino acid sequences; and the like.

Where the amino acid sequence is inserted, deleted or substituted as described above, the position of its insertion, deletion or substitution is not particularly limited.

The proteins used in the present invention are represented in accordance with the conventional way of describing peptides, that is, the N-terminus (amino terminus) at the left hand and the C-terminus (carboxyl terminus) at the right hand. In the protein used in the present invention including the protein comprising the amino acid sequence represented by SEQ ID NO: 1, the C-terminus may be in any form of a carboxyl group (-COOH), a carboxylate ($-COO^-$), an amide ($-CONH_2$) and an ester (-COOR).

Herein, examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl such as a phenyl-$C_{1-2}$ alkyl group, e.g., benzyl, phenethyl, etc.; an α-naphthyl-$C_{1-2}$ alkyl group such as α-naphthylmethyl, etc.; pivaloyloxymethyl and the like.

Where the protein used in the present invention contains a carboxyl group (or a carboxylate) at a position other than the C-terminus, the carboxyl group may be amidated or esterified and such an amide or ester is also included within the protein of the present invention. Examples of the ester group in this case may be the C-terminal esters described above, etc.

Furthermore, examples of the protein used in the present invention include variants wherein the amino group at the N-terminal amino acid residues (e.g., methionine residue) is protected with a protecting group (e.g., a $C_{1-6}$ acyl group such as a $C_{1-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{1-6}$ alkanoyl group, e.g., formyl group, acetyl group, etc.), or conjugated proteins such as glycoproteins having sugar chains; etc.

Specific examples of the protein used in the present invention are a protein comprising the amino acid sequence represented by SEQ ID NO: 1, a protein comprising the amino acid sequence represented by SEQ ID NO: 3, a protein comprising the amino acid sequence represented by SEQ ID NO: 11, and the like.

The partial peptide of the protein used in the present invention may be any peptide as long as it is a partial peptide of the protein used in the present invention described above and preferably has the property equivalent to that of the protein used in the present invention described above.

Specifically, for the purpose of producing the antibody of the present invention described below, there are used peptides containing the sequence of at least 20, preferably at least 50, more preferably at least 70, much more preferably at least 100, and most preferably at least 200 amino acids in the amino acid sequence represented by SEQ ID NO: 1, SEQ ID NO: 3 or SEQ ID NO: 11, etc.

The partial peptides used in the present invention may be those wherein at least 1 or 2 (preferably about 1 to about 10 and more preferably several (1 to 5)) amino acids may be deleted of the amino acid sequence; wherein at least 1 or 2 (preferably about 1 to about 20, more preferably about 1 to about 10 and most preferably several (1 to 5)) amino acids may be added to the amino acid sequence; wherein at least 1 or 2 (preferably about 1 to about 20, more preferably about 1 to about 10 and most preferably several (1 to 5)) amino acids may be inserted in the amino acid sequence; or wherein at least 1 or 2 (preferably about 1 to about 10, more preferably several and most preferably about 1 to about 5) amino acids in the amino acid sequence may be substituted by other amino acids.

In the partial peptide used in the present invention, the C-terminus may be in any form of a carboxyl group (-COOH), a carboxylate ($-COO^-$), an amide ($-CONH_2$) or an ester (-COOR).

In addition, the partial peptide used in the present invention includes variants having a carboxyl group (or a carboxylate) at a position other than the C-terminus, those wherein the amino group at the N-terminal amino acid residues (e.g., methionine residue) is protected with a protecting group; those wherein the N-terminal region is cleaved in vivo and the glutamyl group thus formed is pyroglutaminated; those wherein a substituent on the side chain of an amino acid in the molecule is protected with a suitable protecting group, or conjugated peptides such as so-called glycopeptides having sugar chains; etc., as in the protein of the present invention described above.

The partial peptide used in the present invention may also be used as an antigen for producing the antibodies.

As salts of the protein or partial peptide used in the present invention, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or partial peptide used in the present invention or salts thereof may be manufactured by publicly known methods used to purify proteins from human or warm-blooded animal cells or tissues described above. Alternatively, they may also be manufactured by culturing transformants containing DNAs encoding these proteins. Furthermore, they may also be manufactured by a modification of the methods for peptide synthesis, which will be later described.

Where these proteins are manufactured from human or mammalian tissues or cells, human or non-human mammalian tissues or cells are homogenized, extracted with an acid or the like, and the extract is purified and isolated by a combination of chromatography techniques such as reverse phase chromatography, ion exchange chromatography, and the like.

To synthesize the protein or partial peptide used in the present invention or its salts, or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc.

Using these resins, amino acids, in which α-amino groups and functional groups on the side chains are appropriately protected, are condensed on the resin in accordance with the sequence of the objective protein according to various condensation methods per se known in the art.

At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, and carbodiimides are particularly employed. Examples of such carbodiimides include DCC, N, N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be appropriately chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid any possible effect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (linear, branched or cyclic alkyl esterification of, e.g., methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower ($C_{1-6}$) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the desired protein or partial peptide, for example, the α-carboxyl group of the carboxy terminal amino acid is first protected by amidation; the peptide (protein) chain is then extended from the amino group side to a desired length. Subsequently, a protein or partial peptide, in which only the protecting group of the N-terminal α-amino group of the peptide chain has been eliminated, and a protein or partial peptide, in which only the protecting group of the C-terminal carboxyl group has been eliminated, are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide used in the present invention or salts thereof can be manufactured by publicly known methods for peptide synthesis, or by cleaving the protein used in the present invention with an appropriate peptidase. For the methods for peptide synthesis, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in 1) to 5) below.

1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: Peptide Gosei-no-Kiso to Jikken (Basics and experiments of peptide synthesis), published by Maruzen Co. (1975)
4) Haruaki Yajima & Shunpei Sakakibara: Seikagaku Jikken Koza (Biochemical Experiment) 1, Tanpakushitsu no Kagaku (Chemistry of Proteins) IV, 205 (1977)
5) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the partial peptide can be converted into an appropriate salt by a publicly known method or its modification; when the partial peptide is obtained in a salt form, it can be converted into a free form or other different salt form by a publicly known method or its modification.

The polynucleotide encoding the protein used in the present invention may be any polynucleotide so long as it contains the base sequence encoding the protein used in the present invention described above. Preferably, the polynucleotide is a DNA. The DNA may also be any one of genomic DNA, genomic DNA library, cDNA derived from the cells or tissues described above, cDNA library derived from the cells or tissues described above and synthetic DNA.

The vector used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

Examples of the DNA encoding the protein used in the present invention may be any one of a DNA comprising the base sequence represented by SEQ ID NO: 2, SEQ ID NO: 4 or SEQ ID NO: 12; or a DNA comprising a base sequence hybridizable to the base sequence represented by SEQ ID NO: 2, SEQ ID NO: 4 or SEQ ID NO: 12 under high stringent conditions and encoding a protein which has the properties of substantially the same nature as those of the protein comprising the amino acid sequence represented by SEQ ID NO: 1, SEQ ID NO: 3 or SEQ ID NO: 11 described above.

As the DNA that is hybridizable to the base sequence represented by SEQ ID NO: 2, SEQ ID NO: 4 or SEQ ID NO: 12 under high stringent conditions, there are employed, for example, DNAs comprising base sequences having at least about 50% homology, preferably at least about 60% homology, more preferably at least about 70% homology, still more preferably at least about 80% homology, much more preferably at least about 90% homology and most preferably at least about 95% homology, to the base sequence represented by SEQ ID NO: 2, SEQ ID NO: 4 or SEQ ID NO: 12; and the like.

Homology of the base sequences can be measured under the following conditions (Expectation value =10; gaps are allowed; filtering = ON; match score = 1; mismatch score = -3) using a homology scoring algorithm NCBI BLAST (National Center for Biotechnology Information Basic Local Alignment Search Tool).

The hybridization can be carried out by publicly known methods or by modifications thereof, for example, by the method described in Molecular Cloning, 2nd ed. (J. Sambrook et al., Cold Spring Harbor Lab. Press, 1989). A commercially available library can also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, there are employed: a DNA comprising the base sequence represented by SEQ ID NO: 2, etc. as the DNA encoding the protein comprising the amino acid sequence represented by SEQ ID NO: 1; a DNA comprising the base sequence represented by SEQ ID NO: 4, etc. as the DNA encoding the protein comprising the amino acid sequence represented by SEQ ID NO: 3; a DNA comprising the base sequence represented by SEQ ID NO: 12, etc. as the DNA encoding the protein comprising the amino acid sequence represented by SEQ ID NO: 11, etc.

The polynucleotide (e.g., DNA) encoding the partial peptide used in the present invention may be any polynucleotide so long as it contains the base sequence encoding the partial peptide used in the present invention described above. The polynucleotide may also be any of genomic DNA, genomic DNA library, cDNA derived from the cells and tissues described above, cDNA library derived from the cells and tissues described above and synthetic DNA.

As the DNA encoding the partial peptide used in the present invention, there are employed, for example, a DNA comprising a part of the DNA having the base sequence represented by SEQ ID NO: 2, SEQ ID NO: 4 or SEQ ID NO: 12, or a DNA comprising a base sequence hybridizable to the base sequence represented by SEQ ID NO: 2, SEQ ID NO: 4 or SEQ ID NO: 12 under high stringent conditions and comprising a part of DNA encoding a protein having the activities of substantially the same nature as those of the protein of the present invention, and the like.

The DNA hybridizable to the base sequence represented by SEQ ID NO: 2, SEQ ID NO: 4 or SEQ ID NO: 12 has the same significance as described above.

Methods for the hybridization and high stringent conditions used are the same as those described above.

For cloning of DNAs that completely encode the protein or partial peptide used in the present invention (hereinafter sometimes merely referred to as the protein of the present invention in describing the cloning of DNAs and their expression), the DNA can be either amplified by PCR using synthetic DNA primers containing a part of the base sequence encoding the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Substitution of the base sequence of DNA can be effected by publicly known methods such as the ODA-LA PCR method, the Gapped duplex method, the Kunkel method, etc., or its modifications, using PCR, a publicly known kit available as Mutan™-super Express Km (manufactured by Takara Bio Inc.) or Mutan™-K (manufactured by Takara Bio Inc.), etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by (i) excising the desired DNA fragment from the DNA encoding the protein of the present invention, and then (ii) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, it is preferred to use CMV (cytomegalovirus) promoter, SRα promoter, etc. Where the host is bacteria of the genus Escherichia, preferred examples of the promoter include trp promoter, lac promoter, recA promoter, $\lambda P_L$ promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector comprising the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, $AH22R^-$, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of Bombyx mori can be used [Maeda et al., Nature, 315, 592 (1985)].

Examples of animal cells include monkey cell COS-7, Vero, Chinese hamster cell CHO (hereinafter referred to as CHO cell), dhfr gene-deficient Chinese hamster cell CHO (hereinafter simply referred to as CHO (dhfr-) cell), mouse L cell, mouse AtT-20, mouse myeloma cell, mouse ATDC5 cell, rat GH3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors comprising the DNAs encoding the protein can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the protein of the present invention can be produced in the transformant, on the cell membrane of the transformant, or outside of the transformant.

The protein of the present invention can be separated and purified from the culture described above by the following procedures.

When the protein of the present invention is extracted from the bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc to produce crude extract of the protein. Thus, the crude extract of the protein can be obtained. The buffer used for the procedures may contain a protein modifier such as urea or guanidine hydrochloride, or a surfactant such as Triton X-100™, etc. When the protein is secreted in the culture broth, the supernatant can be separated, after completion of the cultivation, from the bacteria or cell to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be subjected to addition of an appropriate modification or removal of a partial polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

The antibodies to the protein or partial peptide used in the present invention, or its salts may be any of polyclonal and monoclonal antibodies, as long as they are capable of recognizing the protein or partial peptide used in the present invention, or its salts.

The antibodies to the protein or partial peptide used in the present invention, or its salts (hereinafter they are sometimes collectively referred to as the protein of the present invention in the description of the antibodies) can be produced by a publicly known method of producing an antibody or antiserum, using the protein of the present invention as an antigen.

[Preparation of monoclonal antibody]

(a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are monkeys, rabbits, dogs, guinea pigs, mice, rats, sheep, goats and fowl, with the use of mice and rats being preferred.

In the preparation of monoclonal antibody-producing cells, a warm-blooded animal, e.g., mice, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, and (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally in 5% $CO_2$. The antibody titer of the culture supernatant of a hybridoma can be determined as in the assay for the antibody titer in antisera described above.

(b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

[Preparation of polyclonal antibody]

The polyclonal antibody of the present invention can be manufactured by publicly known methods or modifications thereof. For example, a warm-blooded animal is immunized with an immunogen (protein antigen) per se, or with a complex of immunogen and a carrier protein formed in a manner similar to the method described above for the manufacture of monoclonal antibodies. The product containing the antibody to the protein of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The antisense polynucleotide having a complementary or substantially complementary base sequence to the base sequence of the polynucleotide encoding the protein or partial peptide used in the present invention (e.g., DNA (hereinafter these DNAs are sometimes collectively referred to as the DNA of the present invention in the description of antisense polynucleotide)) can be any antisense polynucleotide, so long as it possesses a base sequence complementary or substantially complementary to the base sequence of the polynucleotide (e.g., DNA) of the present invention and capable of suppressing the expression of said DNA, but antisense DNA is preferred.

The base sequence substantially complementary to the DNA of the present invention may include, for example, a base sequence having at least about 70% homology, preferably at least about 80% homology, more preferably at least about 90% homology and most preferably at least about 95% homology, to the entire base sequence or to its partial base sequence (i.e., complementary strand to the DNA of the present invention), and the like. Especially in the entire base sequence of the complementary strand to the DNA of the present invention, preferred are (i) an antisense polynucleotide having at least about 70% homology, preferably at least about 80% homology, more preferably at least about 90% homology and most preferably at least about 95% homology, to the complementary strand of the base sequence which encodes the N-terminal region of the protein of the present invention (e.g., the base sequence around the initiation codon) in the case of antisense polynucleotide directed to translation inhibition and (ii) an antisense polynucleotide having at least about 70% homology, preferably at least about 80% homology, more preferably at least about 90% homology and most preferably at least about 95% homology, to the complementary strand of the entire base sequence of the DNA of the present invention having intron, in the case of antisense polynucleotide directed to RNA degradation by RNaseH, respectively.

Specific examples include an antisense polynucleotide containing the entire or part of a base sequence complementary or substantially complementary to a base sequence of DNA containing the base sequence represented by SEQ ID NO: 2, SEQ ID NO: 4 or SEQ ID NO: 12, preferably an antisense polynucleotide containing the entire or part of a base sequence complementary to a base sequence of DNA containing the base sequence represented by SEQ ID NO: 2, SEQ ID NO: 4 or SEQ ID NO: 12, etc.

The antisense polynucleotide is generally constituted by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. Also, the sugar (deoxyribose) in each nucleotide may be replaced by a chemically modified structure such as 2'-O-methylation, etc. The base part (pyrimidine, purine) may also be chemically modified and may be any one which hybridizes to a DNA containing the base sequence represented by SEQ ID NO: 2 or SEQ ID NO: 4, etc. These antisense polynucleotides may be synthesized using a publicly known DNA synthesizer, etc.

According to the present invention, the antisense polynucleotide (nucleic acid) capable of inhibiting the replication or expression of a gene for the protein of the present invention can be designed and synthesized based on the base sequence information of cloned or identified protein-encoding DNA. Such a polynucleotide (nucleic acid) is hybridizable to RNA of a gene for the protein of the present invention to inhibit the synthesis or function of said RNA or is capable of modulating and/or controlling the expression of a gene for the protein of the present invention via interaction with RNA associated with the protein of the present invention. Polynucleotides complementary to the selected sequences of RNA associated with the protein of the present invention and polynucleotides specifically hybridizable to RNA associated with the protein of the present invention are useful in modulating/controlling the in vivo and in vitro expression of the protein gene of the present invention, and are useful for the treatment or diagnosis of diseases, etc. The term "corresponding" is used to mean homologous to or complementary to a particular sequence of the nucleotide including the gene, base sequence or nucleic acid. The term "corresponding" between nucleotides, base sequences or nucleic acids and peptides (proteins) usually refer to amino acids of a peptide (protein) under the order derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the targeted nucleic acids and the polynucleotides complementary to at least a part of the target region, specifically the relationship between the target nucleic acids and the polynucleotides hybridizable to the target region, can be denoted to be "antisense." Examples of the antisense polynucleotides include polydeoxyribonucleotides containing 2-deoxy-D-ribose, polynucleotides containing D-ribose, polynucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA: RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, enhancing the cell permeability of the antisense nucleic acid, increasing the affinity of the nucleic acid to the targeted sense strand to a higher level, or minimizing the toxicity, if any, of the antisense nucleic acid.

Most of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993;

etc.

The antisense polynucleotide of the present invention may contain altered or modified sugars, bases or linkages. The antisense polynucleotide may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense polynucleotide can be examined using the transformant of the present invention, the gene expression system of the present invention in vivo and in vitro, or the translation system for the protein of the present invention in vivo and in vitro. The nucleic acid can be applied to cells by a variety of publicly known methods.

Hereinafter, the protein of the present invention, its partial peptides, or salts thereof (hereinafter sometimes merely referred to as the protein of the present invention), the DNA encoding the protein of the present invention or its partial peptides (hereinafter sometimes merely referred to as the DNA of the present invention), the antibodies to the protein of the present invention, its partial peptides, or salts thereof (hereinafter sometimes merely referred to as the antibody of the present invention) and the antisense polynucleotides to the DNA of the present invention (hereinafter sometimes merely referred to as the antisense polynucleotide of the present invention) are specifically described for their applications.

The protein of the present invention is increasingly expressed in arteriosclerotic lesions and thus available as a diagnostic marker for arteriosclerosis. The protein of the present invention is also available as a diagnostic marker for diabetic complications since D-PDMP, which is the glucosylceramide synthase or by lactosylceramide synthase inhibitor, suppresses the action of TNF-$\alpha$ that inhibits phosphorylation of IRS-1 in response to insulin stimulation.

The protein of the present invention is useful as a maker in early diagnosis of arteriosclerotic disorders or diabetic complications, or in predicting the progression of arteriosclerosis or the progression of diabetes mellitus.

Moreover, the medicaments comprising the antisense polynucleotide of a gene encoding the protein of the present invention, siRNA or shRNA for a gene encoding the protein of the present invention, the compound or its salt that inhibits the activity of the protein of the present invention, the compound or its salt that inhibits the expression of a gene for the protein of the present invention, the compound or its salt that inhibits the production of the protein of the present invention, the aptamer to the protein of the present invention or the antibody to the protein of the present invention, etc. can be used as, for example, medicaments for preventing/treating arteriosclerosis, agents for preventing/treating arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.), and the like.

In addition, the glucosylceramide or lactosylceramide synthesized by the protein of the present invention, or GM3 can be used as a diagnostic marker for arteriosclerosis or diabetes mellitus. In other words, they are useful as markers in early diagnosis of arteriosclerotic disorders or diabetic complications, or in predicting the progression of arteriosclerosis.

### (1) Screening of drug candidate compound for disease

The protein of the present invention shows enhanced expression in arteriosclerotic lesions. In addition, arteriosclerotic lesions are improved when the protein of the present invention is inhibited ["the activity of the protein of the present invention is inhibited," "the expression of a gene for the protein of the present invention is inhibited" or "the production of the protein of the present invention is inhibited" are sometimes collectively referred to as "the protein of the present invention is inhibited;" also, the compound or its salt per se that inhibits the protein of the present invention is sometimes referred to as the inhibitor of the protein of the present invention; furthermore, the medicament of the present invention for preventing/treating arteriosclerosis includes the compound or its salt per se that inhibits the protein of the present invention, pharmaceutical compositions comprising the same, and so on]. Besides, D-PDMP, which is the inhibitor of glucosylceramide synthase or lactosylceramide synthase, suppresses the action of TNF-$\alpha$ that inhibits

phosphorylation of IRS-1 in response to insulin stimulation.

The glucosylceramide synthase, lactosylceramide synthase and GM3 synthase are the enzymes involved in the ganglioside biosynthesis system. The glucosylceramide synthase, lactosylceramide synthase and GM3 synthase are enzymes which synthesize glucosylceramide from ceramide, lactosylceramide from glucosylceramide and GM3 from lactosylceramide, respectively. In other words, the glucosylceramide synthase is the enzyme located upstream of GM3 synthase in the ganglioside biosynthesis system. The glucosylceramide synthase activity is associated with regulation of the GM3 contents in tissues, which examples are found in the following reports. When lipopolysaccharides are administered to hamsters, the expression level of glucosylceramide synthase increased in the liver and the GM3 level in the liver also increased with an increase of the glucosylceramide synthase activity (J. Biol. Chem., 274, 19707-19713, 1999). Also when TNF-α or IL-1β is administered, the expression level of glucosylceramide synthase increased so that similar effects can be expected. Further in 3T3-L1 adipocytes, the expression of GM3 synthase increased in response to TNF-α stimulation and the GM3 level increased as well (J. Biol. Chem., 277, 3085-3092, 2002). In other words, the glucosylceramide synthase, lactosylceramide synthase and GM3 synthase are all regulated on their expression levels in regulating the GM3 level in cells or tissues, as in the case of, e.g., TNF-α stimulation.

Accordingly, the compound or its salts that inhibits the protein of the present invention can be used as, for example, a medicament for preventing/treating arteriosclerosis, an agent for preventing/treating arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.), and the like.

Accordingly, the protein of the present invention is useful as a reagent for screening the compound or its salts that inhibits the activity of the protein of the present invention.

Specific examples of the screening method used include the following features, and so on.

(1a) A method of screening the compound or its salts that inhibits the activity of protein A of the present invention, which comprises comparing (i) the glucosylceramide synthase activity in cells capable of producing the activity of protein A of the present invention, their cell extracts or purified proteins and (ii) the glucosylceramide synthase activity in cells capable of producing the protein A of the present invention, or their cell extracts or purified protein, in the presence of a test compound.

(1b) A method of screening the compound or its salts that inhibits the activity of protein B of the present invention, which comprises comparing (i) the lactosylceramide synthase activity in cells capable of producing the activity of protein B of the present invention, their cell extracts or purified protein and (ii) the lactosylceramide synthase activity in cells capable of producing the protein B of the present invention, or their cell extracts or purified protein, in the presence of a test compound.

(1c) A method of screening the compound or its salts that inhibits the activities of proteins A and B of the present invention, which comprises comparing (i) the glucosylceramide synthase activity and the lactosylceramide synthase activity in cells capable of producing the proteins A and B of the present invention, their cell extracts or purified proteins and (ii) the glucosylceramide synthase activity and the lactosylceramide synthase activity in cells capable of producing the proteins A and B of the present invention, or their cell extracts or purified proteins, in the presence of a test compound.

(1d) A method of screening the compound or its salts that inhibits the activities of proteins A and C of the present invention, which comprises comparing (i) the glucosylceramide synthase activity and the GM3 synthase activity in cells capable of producing the proteins A and C of the present invention, their cell extract or purified proteins and (ii) the glucosylceramide synthase activity and the GM3 synthase activity in cells capable of producing the proteins A and C of the present invention, or their cell extracts or purified proteins, in the presence of a test compound.

(1e) A method of screening the compound or its salts that inhibits the activities of proteins B and C of the present invention, which comprises comparing (i) the lactosylceramide synthase activity and the GM3 synthase activity in cells capable of producing the proteins B and C of the present invention, their cell extracts or purified proteins and (ii) the lactosylceramide synthase activity and the GM3 synthase activity in cells capable of producing the proteins B and C of the present invention, or their cell extracts or purified proteins, in the presence of a test compound.

(1f) A method of screening the compound or its salts that inhibits the activities of proteins B and C of the present invention, which comprises comparing (i) the glucosylceramide synthase activity, the lactosylceramide synthase activity and the GM3 synthase activity in cells capable of producing the proteins A, B and C of the present invention, their cell extracts or purified proteins and (ii) the glucosylceramide synthase activity, the lactosylceramide synthase activity and the GM3 synthase activity in cells capable of producing the proteins A, B and C of the present invention, or their cell extracts or purified proteins, in the presence of a test compound.

(2a) A method of screening the compound or its salts that inhibits the activity of protein A of the present invention, which comprises comparing (iii) the lipid accumulation promoting activity of protein A of the present invention and (iv) the lipid accumulation promoting activity of a mixture of the protein A of the present invention and a test compound.
(2b) A method of screening the compound or its salts that inhibits the activity of protein B of the present invention, which comprises comparing (iii) the lipid accumulation promoting activity of protein B of the present invention and (iv) the lipid accumulation promoting activity of a mixture of the protein B of the present invention and a test compound.
(2c) A method of screening the compound or its salts that inhibits the activities of proteins A and B of the present invention, which comprises comparing (iii) the lipid accumulation promoting activities of proteins A and B of the present invention and (iv) the lipid accumulation promoting activities of a mixture of the proteins A and B of the present invention and a test compound.
(2d) A method of screening the compound or its salts that inhibits the activities of proteins A and C of the present invention, which comprises comparing (iii) the lipid accumulation promoting activities of proteins A and C of the present invention and (iv) the lipid accumulation promoting activities of a mixture of the proteins A and C of the present invention and a test compound.
(2e) A method of screening the compound or its salts that inhibits the activities of proteins B and C of the present invention, which comprises comparing (iii) the lipid accumulation promoting activities of proteins B and C of the present invention and (iv) the lipid accumulation promoting activities of a mixture of the proteins B and C of the present invention and a test compound.
(2f) A method of screening the compound or its salts that inhibits the activities of proteins A, B and C of the present invention, which comprises comparing (iii) the lipid accumulation promoting activities of proteins A, B and C of the present invention and (iv) the lipid accumulation promoting activities of a mixture of the proteins A, B and C of the present invention and a test compound.

As the cells capable of producing the protein of the present invention, there are used, for example, hosts (transformants) transformed by vectors comprising the DNA encoding the protein of the present invention described above. Examples of the hosts, which are preferably used, are animal cells such as COS7 cells, CHO cells, HEK293 cells, etc. For the screening, preferably used are transformants in which the protein of the present invention is expressed intracellularly or extracellularly, e.g., by incubation according to the procedure described above. The procedure for incubation of the cells capable of expressing the protein of the present invention is the same as the incubation of transformants of the present invention described above.

The test compounds may be peptides, proteins, antibodies, non-peptide compounds, synthetic compounds, fermentation products, cell extract, plant extract, animal tissue extract, plasma. The test compounds may form salts and as salts of the test compounds, there are, for example, metal salts, ammonium salts, salts with organic bases, salts with inorganic acids, salts with organic acids, salts with basic or acidic amino acids, etc. Preferred examples of the metal salts include alkali metal salts such as sodium salts, potassium salts, etc.; alkaline earth meal salts such as calcium salts, magnesium salts, barium salts, etc.; aluminum salts, etc. Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine,
N,N'-dibenzylethylenediamine, etc. Preferred examples of the salts with inorganic acids include salts with hydrochloric acid, hydrobromic acid, nitric acid, sulfuric acid, phosphoric acid, etc. Preferred examples of the salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Preferred examples of the salts with basic amino acids include salts with arginine, lysine, ornithine, etc., and preferred examples of the salts with acidic amino acids include salts with aspartic acid, glutamic acid, etc.

Among these salts, preferred are pharmaceutically acceptable salts. Where the compounds have, for example, acidic functional groups in the compound, the salts include inorganic salts such as alkali metal salts (e.g., sodium salts, potassium salts, etc.), alkaline earth metal salts (e.g., calcium salts, magnesium salts, barium salts, etc.), etc.; ammonium salts, etc.; and where the compounds have basic functional groups in the compound, the salts include salts with inorganic acids such as hydrobromic acid, nitric acid, sulfuric acid, phosphoric acid, etc., or salts with organic acids such as acetic acid, phthalic acid, fumaric acid, oxalic acid, tartaric acid, maleic acid, citric acid, succinic acid, methanesulfonic acid, p-toluenesulfonic acid, etc.

For example, when a test compound inhibits each activity in the case (ii) described above by at least about 20%, preferably at least 30% and more preferably at least about 50%, as compared to the case (i) above, the test compound can be selected as the compound that inhibits the activity of the protein of the present invention.

For example, when a test compound inhibits the lipid accumulation promoting activity in the case (iv) described above by at least about 20%, preferably at least 30% and more preferably at least about 50%, as compared to the case (iii) above, the test compound can be selected as the compound that inhibits the activity of the protein of the present invention.

The compound that has the activity of inhibiting the activity of the protein of the present invention is useful as a safe and low toxic medicament for inhibiting physiological activities of the protein of the present invention.

The compound or its salt, which is obtained by using the screening method or screening kit of the present invention, is a compound selected from, for example, peptides, proteins, antibodies, non-peptide compounds, synthetic compounds, fermentation products, cell extracts, plant extracts, animal tissue extracts, blood plasma, etc. The salts of these compounds used are those given above as salts of the test compounds.

Moreover, the expression of genes for the protein of the present invention is also increased in arteriosclerotic lesions. By inhibiting the expression of these genes, arteriosclerotic lesions are improved and thus, the compound or its salt that inhibits the expression of genes for the protein of the present invention can also be used as, for example, a medicament for preventing/treating arteriosclerosis, an agent for preventing/treating arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.), and the like.

Therefore, the polynucleotide (e.g., DNA) of the present invention is useful as a reagent for screening the compound or its salt that inhibits the expression of genes for the protein of the present invention.

The screening method includes a screening method which comprises comparing (v) the case where a cell capable of producing the protein of the present invention is cultured and (vi) the case where a cell capable of producing the protein of the present invention is cultured, in the presence of a test compound.

In the method described above, expression levels of the gene (specifically, levels of the protein of the present invention or levels of the polynucleotide encoding the protein (e.g., mRNA, etc.)) in the cases (v) and (vi) are assayed and compared.

(3 a) A method of screening the compound or its salt that inhibits the expression of a gene for protein A of the present invention, which comprises assaying expression levels of the gene for protein A (v) in the case where cells capable of producing protein A of the present invention are cultured and (vi) in the case where cells capable of producing protein A of the present invention are cultured in the presence of a test compound, respectively, and comparing the expression levels.

(3b) A method of screening the compound or its salt that inhibits the expression of a gene for protein B of the present invention, which comprises assaying expression levels of the gene for protein B (v) in the case where cells capable of producing protein B of the present invention are cultured and (vi) in the case where cells capable of producing protein B of the present invention are cultured in the presence of a test compound, respectively, and comparing the expression levels.

(3c) A method of screening the compound or its salt that inhibits the expression of a gene for protein A of the present invention and the expression of a gene for protein B of the present invention, which comprises assaying expression levels of the genes for proteins A and B (v) in the case where cells capable of producing proteins A and B of the present invention are cultured and (vi) in the case where cells capable of producing proteins A and B of the present invention are cultured in the presence of a test compound, respectively, and comparing the expression levels.

(3d) A method of screening the compound or its salt that inhibits the expression of a gene for protein A of the present invention and the expression of a gene for protein C of the present invention, which comprises assaying expression levels of the genes for proteins A and C (v) in the case where cells capable of producing proteins A and C of the present invention are cultured and (vi) in the case where cells capable of producing proteins A and C of the present invention are cultured in the presence of a test compound, respectively, and comparing the expression levels.

(3e) A method of screening the compound or its salt that inhibits the expression of a gene for protein B of the present invention and the expression of a gene for protein C of the present invention, which comprises assaying expression levels of the genes for proteins B and C (v) in the case where a cell capable of producing proteins B and C of the present invention is cultured and (vi) in the case where a cell capable of producing proteins B and C of the present invention is cultured in the presence of a test compound, respectively, and comparing the expression levels.

(3f) A method of screening the compound or its salt that inhibits the expression of a gene for protein A of the present invention, the expression of a gene for protein B of the present invention and the expression of a gene for protein C of the present invention, which comprises assaying expression levels of the genes for proteins A, B and C (v) in the case where a cell capable of producing proteins A, B and C of the present invention is cultured and (vi) in the case where a cell capable of producing proteins A, B and C of the present invention is cultured in the presence of a test compound, respectively, and comparing the expression levels.

Examples of the test compounds and the cells capable of producing the protein of the present invention are the same as those described above.

The level of the protein can be determined by publicly known methods, e.g., by measuring the aforesaid protein present in the cell extract, etc., using an antibody capable of recognizing the protein of the present invention, in accordance with methods like western blot analysis, ELISA, etc., or their modifications.

The mRNA level can be determined by publicly known methods, e.g., in accordance with methods such as northern hybridization using a nucleic acid comprising the entire or a part of SEQ ID NO: 2, SEQ ID NO: 4 or SEQ ID NO: 12 as a probe, or PCR using a nucleic acid comprising the entire or a part of SEQ ID NO: 2, SEQ ID NO: 4 or SEQ ID NO: 12 as a primer, or modifications thereof.

For example, when a test compound inhibits the expression of the gene in the case (iv) described above by at least about 20%, preferably at least 30% and more preferably at least about 50%, as compared to the case (v) above, the test compound can be selected to be the compound that inhibits the expression of the gene for the protein of the present invention.

(i) The compound or its salt that inhibits the activity of the protein of the present invention, (ii) the compound or its salt that inhibits the expression of a gene for the protein of the present invention and (iii) the compound or its salt that inhibits the production of the protein of the present invention preferably have, e.g., effects of promoting the cholesterol efflux activity, effects of inhibiting the lipoprotein uptake activity.

The cholesterol efflux activity is assayed by publicly known methods, e.g., by the method described in J. Biol. Chem., 276, 43564-43569, 2001, etc., or its modifications.

Cells (e.g., macrophages, etc.) are cultured in a medium supplemented with radioisotope (e.g., [$^3$H], [$^{14}$C], etc.)-labeled cholesterol or cholesterol ester and washed with a buffer (e.g., bovine serum albumin-containing phosphate buffer, phosphate buffer, etc.) to remove the radioisotope not taken up into the cells. The cells described above are cultured in the presence of the compounds (i) to (iii) described above and then brought in contact with high density lipoprotein (HDL), apolipoprotein A1, cyclodextrins, etc. (e.g., exchange of medium containing HDL, apolipoprotein A1 or cyclodextrins, followed by culturing) to assay the radioactivity. The radioactivity in the cell lysate is assayed as well to determine what percentage of the radioisotope taken up is effluxed into the culture supernatant.

The lipoprotein uptake activity is assayed by publicly known methods, e.g., by the method described in Pro. Natl. Acad. Sci., 88, 4931-4935, 1991, etc., or its modifications.

In the presence of the compounds (i) to (iii) described above, cells are cultured in a medium containing lipoproteins (e.g., low density lipoprotein, denatured low density lipoprotein, etc.) labeled with radioisotopes (e.g., [$^{125}$I], [$^{131}$I], [$^3$H], [$^{14}$C], [$^{32}$P], [$^{33}$P], [$^{35}$S], etc.) and then washed with buffer to assay the radioactivity taken up with a scintillation counter, etc.

In the presence of the compounds (i) to (iii) described above, cells are cultured in a medium containing lipoproteins (e.g., low density lipoprotein, denatured low density lipoprotein, etc.) labeled with fluorescent substances [e.g., cyanine fluorescent substances (e.g., Cy2, Cy3, Cy5, Cy5.5, Cy7 (manufactured by Amersham Biosciences), etc.), fluorescamine, fluorescein isothiocyanate, etc.], and washed with a buffer (e.g., a phosphate buffer, etc.). Thereafter, the fluorescent activity is measured using a fluorescence plate reader. Alternatively, the cells are detached and the fluorescent substance-labeled lipoprotein which is taken up is assayed by FACS.

The screening kit of the present invention comprises the polynucleotide encoding the protein used in the present invention, its partial peptide or a salt thereof, or the cells capable of producing the protein used in the present invention or its partial peptide.

The compound or its salt, which is obtained using the screening method or screening kit of the present invention, is the compound or its salt selected from the test compounds described above (e.g., peptides, proteins, antibodies, non-peptide compounds, synthetic compounds, fermentation products, cell extracts, plant extracts, animal tissue extracts, blood plasma, etc.), and is the compound or its salt that inhibits the activities (e.g., the glucosylceramide synthase activity, the lactosylceramide synthase activity, the GM3 synthase activity, etc.) of the protein of the present invention, the compound or its salt that inhibits the expression of a gene for the protein of the present invention, or the compound or its salt that inhibits the production of the protein of the present invention.

The salts of these compounds used are the same as the salts of the test compound described above.

The compound or its salt that inhibits the activity of the protein of the present invention, the compound or its salt that inhibits the expression of a gene for the protein of the present invention, and the compound or its salt that inhibits the production of the protein of the present invention are useful as, for example, medicaments for preventing/treating arteriosclerosis, agents for preventing/treating arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.), and the like.

The compound or its salt that inhibits the activity of the protein of the present invention, the compound or its salt that inhibits the expression of a gene for the protein of the present invention, the compound or its salt that inhibits the production of the protein of the present invention and the antibody of the present invention can also be used in combination of two or more with each other and as agents for preventing/treating the diseases described above.

Where the compound or its salt obtained by using the screening method or screening kit of the present invention is used as the preventive/therapeutic agent described above, the compound can be prepared into pharmaceutical preparations in a conventional manner.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, intraarticular injections, etc. These injectable preparations may be prepared by methods publicly known. For example, the injectable preparations may be prepared by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the aforesaid antibody or its salt with conventional bases for suppositories.

Advantageously, the oral or parenteral pharmaceutical compositions described above are prepared into pharmaceutical preparations with a unit dose suitable for a dose of the active ingredient. Such unit dose preparations include, for example, tablets, pills, capsules, injections (ampoules), suppositories, etc. The amount of the aforesaid compound contained is generally 5 to 500 mg per dosage unit form; it is preferred that the aforesaid antibody is contained in about 5 to about 100 mg especially in the form of injection, and in 10 to 250 mg for the other forms.

Each of the compositions described above may further contain other active components unless formulation causes any adverse interaction with the compound described above.

Since the pharmaceutical preparations thus obtained are safe and low toxic, they can be administered to human or warm-blooded animal (e.g., mouse, rat, rabbit, sheep, swine, bovine, horse, fowl, cat, dog, monkey, chimpanzee, etc.) orally or parenterally.

The dose of the above compound or its salt may vary depending upon its effect, target disease, subject to be administered, conditions, route of administration, etc. For example, when the compound or its salt that inhibits the activity of the protein of the present invention is orally administered for the purpose of treating, for example, arteriosclerotic disease (e.g., myocardial infarction, unstable angina, etc.), the compound or its salt is generally administered to an adult (as 60 kg body weight) in a daily dose of about 0.1 to about 100 mg, preferably about 1.0 to about 50 mg and more preferably about 1.0 to about 20 mg. In parenteral administration, a dose of the said compound or its salt may vary depending upon target disease, subject to be administered, conditions, route of administration, etc. When the compound or its salt that inhibits the activity of the protein of the present invention is administered to an adult (as 60 kg body weight) in the form of an injectable preparation for the purpose of treating, for example, arteriosclerotic disease (e.g., myocardial infarction, unstable angina, etc.), it is advantageous to administer the compound or its salt by way of injection in a daily dose of about 0.01 to about 30 mg, preferably about 0.1 to about 20 mg, and more preferably about 0.1 to about 10 mg. For other animal species, the corresponding dose as converted per 60 kg weight can be administered.

The compound or its salt that inhibits the expression of a gene for the protein of the present invention, the compound or its salt that inhibits the production of the protein of the present invention, and the like can be administered as in the compound or its salt that inhibits the activity of the protein of the present invention.

**(2) Quantification of the protein of the present invention, its partial peptide or its salt**

The antibody to the protein of the present invention (hereinafter sometimes merely referred to as the antibody of the present invention) is capable of specifically recognizing the protein of the present invention and therefore can be used for quantification of the protein of the present invention in a test sample fluid, in particular, for quantification by sandwich immunoassay; etc.

That is, the present invention provides:

(i) a method of quantifying the protein of the present invention in a test sample fluid, which comprises competitively reacting the antibody of the present invention, a test sample fluid and a labeled form of the protein of the present invention, and measuring the proportion of the labeled protein of the present invention bound to said antibody; and,
(ii) a method of quantifying the protein of the present invention in a test sample fluid, which comprises reacting a test sample fluid simultaneously or continuously with the antibody of the present invention immobilized on a carrier and another labeled antibody of the present invention, and then measuring the activity of the labeling agent on the insoluble carrier.

In the quantification method (ii) described above, it is preferred that one antibody is capable of recognizing the N-terminal region of the protein of the present invention, while another antibody is capable of reacting with the C-terminal region of the protein of the present invention.

The monoclonal antibody to the protein of the present invention (hereinafter sometimes referred to as the monoclonal antibody of the present invention) can be used to quantify the protein of the present invention. In addition, the protein can be detected by means of a tissue staining as well. For these purposes, the antibody molecule per se may be used or F $(ab')_2$, Fab' or Fab fractions of the antibody molecule may also be used.

The method of quantifying the protein of the present invention using the antibody of the present invention is not to be particularly limited. Any quantification method can be used, so long as the amount of antibody, antigen or antibody-antigen complex corresponding to the amount of antigen (e.g., the amount of the protein) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use the sandwich method described later.

Examples of the labeling agent available for the assay method using the labeling substance are radioisotopes (e.g., [$^{125}I$], [$^{131}I$], [$^{3}H$], [$^{14}C$], [$^{32}P$], [$^{33}P$], [$^{35}S$], etc.), fluorescent substances (e.g., cyanine fluorescent substances [e.g., Cy2, Cy3, Cy5, Cy5.5, Cy7 (Amersham Biosciences)), fluorescamine, fluorescein isothiocyanate], enzymes (e.g., β-galactosidase, β-glucosidase, alkaline phosphatase, peroxidase, malate dehydrogenase), luminescent substances (e.g., luminol, luminal derivatives, luciferin, lucigenin), lanthanide elements, etc.

For immobilization of the antigen or antibody, physical adsorption may be used. Chemical binding techniques conventionally used for insolubilization or immobilization of proteins, enzymes, etc. may also be used. For carriers, there are used, e.g., insoluble polysaccharides such as agarose, dextran, cellulose, etc.; synthetic resin such as polystyrene, polyacrylamide, silicon, etc., glass and the like.

In the sandwich method, the immobilized monoclonal antibody of the present invention is reacted with a test fluid (primary reaction), then with a labeled form of another monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method of the present invention, antibodies that bind to different sites of the protein of the present invention are preferably used as the monoclonal antibodies of the present invention used for the primary and secondary reactions. That is, in the antibodies used for the primary and secondary reactions are, for example, when the antibody used in the secondary reaction recognizes the C-terminal region of the protein of the present invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, the competitive method, the immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying each of these immunological methods to the quantification method of the present invention, any particular conditions or procedures are not required. The assay system for the protein of the present invention or its salt is constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing), etc.

As described above, the protein of the present invention can be quantified with high sensitivity, using the antibody of the present invention.

Furthermore, when an increased level of the protein of the present invention is detected by quantifying the level of the protein of the present invention using the antibody of the present invention, it can be diagnosed that one suffers from, for example, arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.) or the like; or it is highly likely to suffer from these diseases in the future.

For example, the diagnosis can be made by:

(a) quantifying the level of protein A of the present invention using the antibody to protein A of the present invention;
(b) quantifying the level of protein B of the present invention using the antibody to protein B of the present invention;
(c) quantifying the levels of proteins A and B of the present invention using the antibody to protein A of the present invention and he antibody to protein B of the present invention;
(d) quantifying the levels of proteins A and C of the present invention using the antibody to protein A of the present invention and the antibody to protein C of the present invention;
(e) quantifying the levels of proteins B and C of the present invention using the antibody to protein B of the present invention and the antibody to protein C of the present invention; or,
(f) quantifying the levels of proteins A, B and C of the present invention using the antibody to protein A of the present invention, the antibody to the protein B of the present invention and the antibody to the protein C of the present invention.

Moreover, the antibody of the present invention can be used to detect the protein of the present invention, which is present in a test sample such as body fluids, tissues, etc. The antibody can also be used to prepare an antibody column for purification of the protein of the present invention, detect the protein of the present invention in each fraction upon purification, analyze the behavior of the protein of the present invention in the cells under investigation; etc.

### (3) Gene diagnostic agent

By using the DNA of the present invention, e.g., as a probe, an abnormality (gene abnormality) of the DNA or mRNA encoding the protein of the present invention or its partial peptide in human or warm-blooded animal (e.g., rat, mouse, guinea pig, rabbit, fowl, sheep, swine, bovine, horse, cat, dog, monkey, chimpanzee, etc.) can be detected. Therefore, the DNA of the present invention is useful as a gene diagnostic agent for detecting damages to the DNA or mRNA, its mutation, or decreased expression, increased expression, overexpression, etc. of the DNA or mRNA, and so on.

The gene diagnosis described above using the DNA of the present invention can be performed by, for example, the publicly known northern hybridization assay or the PCR-SSCP assay (Genomics, 5, 874-879 (1989); Proceedings of the National Academy of Sciences of the United States of America, 86, 2766-2770 (1989)), etc.
Where an increased expression is detected by, e.g., northern hybridization or DNA mutation is detected by the PCR-SSCP assay, it can be diagnosed that it is highly likely to suffer from for example, arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.) or the like.
For, example, the diagnosis can be made by:

(a) quantifying the expression level of a gene for protein A of the present invention using the polynucleotide encoding protein A of the present invention;
(b) quantifying the expression level of a gene for protein B of the present invention using the polynucleotide encoding protein B of the present invention;
(c) quantifying the expression levels of genes for proteins A and B of the present invention using the polynucleotide encoding protein A of the present invention and the polynucleotide encoding protein B of the present invention;
(d) quantifying the expression levels of genes for proteins A and C of the present invention using the polynucleotide encoding protein A of the present invention and the polynucleotide encoding protein C of the present invention;
(e) quantifying the expression levels of genes for proteins B and C of the present invention using the polynucleotide encoding protein B of the present invention and the polynucleotide encoding protein C of the present invention; or,
(f) quantifying the expression levels of genes for proteins A, B and C of the present invention using the polynucleotide encoding protein A of the present invention, the polynucleotide encoding protein B of the present invention and the polynucleotide encoding protein C of the present invention.

**(4) Medicament comprising antisense polynucleotide**

The antisense polynucleotide of the present invention that binds to the DNA of the present invention complementarily to inhibit expression of the DNA is low toxic and can suppress the functions of the protein of the present invention or the DNA of the present invention in vivo. Thus, the antisense polynucleotide can be used as a medicament such as an agent for preventing/treating, for example, arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.) or the like.
Where the antisense polynucleotide described above is used as a medicament, it can be prepared into pharmaceutical preparations by publicly known methods, and the preparations can be provided for administration.
For example, when the antisense polynucleotide described above is used, the antisense polynucleotide alone is administered directly, or the antisense polynucleotide is inserted into an appropriate vector such as retrovirus vector, adenovirus vector, adenovirus-associated virus vector, etc., followed by treating in a conventional manner. The antisense polynucleotide may then be administered orally or parenterally to human or a mammal (e.g., rat, rabbit, sheep, swine, bovine, cat, dog, monkey, etc.) in a conventional manner. The antisense polynucleotide may also be administered as it stands, or may be prepared in pharmaceutical preparations together with a physiologically acceptable carrier to assist its uptake, which are then administered by gene gun or through a catheter such as a catheter with a hydrogel. Alternatively, the antisense polynucleotide may be prepared into an aerosol, which is topically administered into the trachea as an inhaler.
Further for the purposes of improving pharmacokinetics, prolonging a half-life and improving intracellular uptake efficiency, the antisense polynucleotide described above is prepared into pharmaceutical preparations (injectable preparations) alone or together with a carrier such as liposome, etc. and the preparations may be administered intravenously, subcutaneously, etc.
A dose of the antisense polynucleotide may vary depending on target disease, subject to be administered, conditions, route for administration, etc. For example, where the antisense polynucleotide of the present invention is administered for the purpose of treatment, the antisense polynucleotide is generally administered to an adult (60 kg body weight) in a daily dose of about 0.1 to 100 mg.

In addition, the antisense polynucleotide may also be used as an oligonucleotide probe for diagnosis to examine the presence of the DNA of the present invention in tissues or cells and states of its expression.

As in the antisense polynucleotide described above, the double-stranded RNA (siRNA (small (short) interfering RNA), shRNA (small (short) hairpin RNA), etc. to the polynucleotide of the present invention) comprising a part of RNA encoding the protein of the present invention, ribozyme comprising a part of RNA encoding the protein of the present invention, etc. can also suppress the expression of a gene for the present invention to suppress the in vivo function of the protein used in the present invention or the DNA used in the present invention, and can be used as a medicament such as an agent for preventing/treating, for example, arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.) or the like.

The double-stranded RNA can be designed based on a sequence of the polynucleotide of the present invention and manufactured by modifications of publicly known methods (e.g., Nature, 411, 494, 2001).

The ribozyme can be designed based on a sequence of the polynucleotide of the present invention and manufactured by modifications of publicly known methods (e.g., TRENDS in Molecular Medicine, 7, 221, 2001). For example, the ribozyme can be manufactured by ligating a publicly known ribozyme to a part of the RNA encoding the protein of the present invention. A part of the RNA encoding the protein of the present invention includes a site (RNA fragment) in the vicinity of the cleavage site on the RNA of the present invention, which may be cleaved by a publicly known ribozyme.

Where the double-stranded RNA or ribozyme described above is used as a medicament, the double-stranded RNA or ribozyme is prepared into pharmaceutical preparations as in the antisense polynucleotide, and the preparations can be provided for administration.

As in the antisense polynucleotide described above, the aptamers against the protein of the present invention, etc. can suppress the activity or function of the protein used in the present invention, and can be used as medicaments such as agents for preventing/treating, for example, arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.) or the like.

The aptamers are prepared by using publicly known methods, for example, the SELEX (systematic evolution of ligands by exponential enrichment) method (Annual Review of Medicine, 56, 555-583, 2005). Structures of aptamers can be determined using publicly known methods; based on the structures, the aptamers are prepared by publicly known methods.

When the aforesaid aptamers are used as pharmaceuticals, the aptamers can be prepared into pharmaceutical preparations as in the antisense polynucleotide, and provided for administration.

**(5) Medicament comprising the antibody of the present invention**

The antibody of the present invention (e.g., neutralizing antibody) can be used as a medicament such as an agent for preventing/treating, for example, arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.) or the like. The antibody molecule itself may be used, or $F(ab')_2$, Fab' or Fab fractions of the antibody molecule may be used as well.

Since the aforesaid preventive/therapeutic agent for diseases comprising the antibody of the present invention are safe and low toxic, they can be administered to human or a mammal (e.g., rat, rabbit, sheep, swine, bovine, cat, dog, monkey, etc.) orally or parenterally (e.g., intravascularly, subcutaneously, etc.) either as liquid preparations as they are or as pharmaceutical compositions of adequate dosage form. Preferably, they can be administered in the form of vaccine in a conventional manner.

The antibody of the present invention may be administered as it is or as an appropriate pharmaceutical composition. The pharmaceutical composition used for the administration may contain the antibody of the present invention or its salt, pharmacologically acceptable carriers, diluents or excipients. Such a pharmaceutical composition is provided in the form of pharmaceutical preparations suitable for oral or parenteral administration.

Examples of the composition for parenteral administration are injectable preparations, suppositories, vaccine, etc. The injectable preparations may include dosage forms such as intravenous, subcutaneous, intracutaneous and intramuscular injections, drip infusions, etc. These injectable preparations may be prepared by methods publicly known. The injectable preparations can be prepared, e.g., by dissolving, suspending or emulsifying the antibody of the present invention or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, for example, sesame oil, soybean oil, etc., which may be used in combination with a solubilizing agent such as benzyl benzoate, benzyl alcohol, etc. The injection thus prepared is preferably filled in an appropriate ampoule. The suppository used for rectal administration may be prepared by blending the aforesaid antibody or its salt with conventional bases for suppositories.

Examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Advantageously, the pharmaceutical compositions for oral or parenteral use described above are prepared into pharmaceutical preparations in a unit dose suitable for the dose of active ingredient. Such unit dose preparations include, for example, tablets, pills, capsules, injections (ampoules), suppositories, etc. The amount of the aforesaid antibody contained is generally about 5 to 500 mg per dosage unit form; especially in the form of injection, it is preferred that the aforesaid antibody is contained in about 5 to 100 mg and in about 10 to 250 mg for the other forms.

The dose for administration of the medicament comprising the antibody of the present invention may vary depending upon subject to be administered, target disease, conditions, route of administration, etc. For example, when the antibody is used for the treatment/prevention in an adult, it is advantageous to intravenously administer the antibody of the present invention in a single dose of about 0.01 to about 20 mg/kg body weight, preferably about 0.1 to about 10 mg/kg body weight and more preferably about 0.1 to about 5 mg/kg body weight approximately 1 to 5 times a day, preferably approximately 1 to 3 times a day. In other parenteral administration and oral administration, the antibody can be administered in a dose corresponding to the dose given above. When conditions are especially severe, the dose may be increased depending on the conditions.

The antibody of the present invention may be administered in itself or in the form of an appropriate pharmaceutical composition. The pharmaceutical composition used for the administration may contain the aforesaid antibody or its salt, pharmacologically acceptable carriers diluents or excipients. Such a composition is provided in the form of pharmaceutical preparations suitable for oral or parenteral administration (e.g., intravascular injection, subcutaneous injection, etc.).

Each composition described above may further contain other active ingredients unless the formulation causes any adverse interaction with the antibody described above.

### (6) "The compound or its salt that inhibits the activity of the protein of the present invention," "the compound or its salt that inhibits the expression of a gene for the protein of the present invention" and "the compound or its salt that inhibits the production of the protein of the present invention" in the present invention

"The compound or its salt that inhibits the activity of the protein of the present invention" may be any compound, as long as it is the compound that inhibits the activity of the protein of the present invention (e.g., peptides, proteins, antibodies, non-peptide compounds, synthetic compounds, fermentation products, cell extracts, plant extracts, animal tissue extracts, blood plasma, etc.), and can be used as a preventive/therapeutic agent or preventive/therapeutic medicament for, e.g., arteriosclerosis, arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.) or the like.

"The compound or its salt that inhibits the expression of a gene for the protein of the present invention" may be any compound, as long as it is the compound that inhibits the expression of a gene for the protein of the present invention (e.g., peptides, proteins, antibodies, non-peptide compounds, synthetic compounds, fermentation products, cell extracts, plant extracts, animal tissue extracts, blood plasma, etc.), and can be used as a preventive/therapeutic agent or preventive/therapeutic medicament for, e.g., arteriosclerosis, arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.) or the like.

"The compound or its salt that inhibits the production of the protein of the present invention" may be any compound, as long as it is the compound that inhibits the production of the protein of the present invention (e.g., peptides, proteins, antibodies, non-peptide compounds, synthetic compounds, fermentation products, cell extracts, plant extracts, animal tissue extracts, blood plasma, etc.), and can be used as a preventive/therapeutic agent or preventive/therapeutic medicament for, e.g., arteriosclerosis, arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.) or the like.

The preventive/therapeutic medicament or the preventive/therapeutic agent includes "the compound or its salt that inhibits the activity of the protein of the present invention" per se, "the compound or its salt that inhibits the expression of a gene for the protein of the present invention" per se, "the compound or its salt that inhibits the production of the protein of the present invention" per se, and pharmaceutical compositions comprising any one of these compounds; etc.

The preventive/therapeutic agent can be prepared as described above.

### (7) DNA transgenic animal

The present invention provides a non-human mammal bearing a DNA encoding the protein of the present invention, which is exogenous (hereinafter simply referred to as the exogenous DNA of the present invention) or its variant DNA (sometimes simply referred to as the exogenous variant DNA of the present invention).

That is, the present invention provides:

(1) A non-human mammal bearing the exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector containing the exogenous DNA of the present invention or its variant DNA and capable of expressing in a mammal; etc.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the micro injection method, the particle gun method, the DEAE-dextran method, etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, sheep, goat, rabbits, dogs, cats, guinea pigs, hamsters, mice, rats, etc. Above all, preferred are rodents, especially mice (e.g., C57B1/6 strain, DBA2 strain, etc. for a pure line and for a cross line, $B6C3F_1$ strain, $BDF_1$ strain $B6D2F_1$ strain, BALB/c strain, ICR strain, etc.), rats (Wistar, SD, etc.) or the like, since they are relatively short in ontogeny and life cycle and are easy in breeding from a standpoint of creating model animals for human disease.

"Mammals" in a recombinant vector that can be expressed in the mammals include the aforesaid non-human mammals, human, etc.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses the abnormal protein of the present invention and exemplified by the DNA that expresses a protein for suppressing the function of the normal protein of the present invention.

The exogenous DNA of the present invention may be any one of those derived from a mammal of the same species as, or a different species from, the mammal as the target animal. In transfecting the DNA of the present invention into the target animal, it is generally advantageous to use the DNA as a DNA construct in which the DNA is ligated downstream a promoter capable of expressing the DNA in the target animal. For example, in the case of transfecting the human DNA of the present invention, a DNA transgenic mammal that highly expresses the DNA of the present invention, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the protein of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression described above include (i) promoters for DNA derived from viruses (e.g., simian virus, cytomegalovirus, Moloney leukemia virus, JC virus, breast cancer virus, poliovirus, etc.), and (ii) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), peptide chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human peptide chain elongation factor 1α (EF-1α) promoters, human and chicken β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal protein obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by crossing.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the exogenous DNA of the present invention is excessively present in all of the germinal cells and somatic cells thereof. The offspring of the animal that inherits the exogenous DNA of the present invention have excessively the exogenous DNA of the present invention in all of the germinal cells and somatic cells thereof.

It is possible to obtain homozygous animals having the transfected DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the protein of the present invention and the pathological mechanism of the disease associated with the protein of the present invention and to investigate how to treat these diseases.

Furthermore, since a mammal transfected with the exogenous normal DNA of the present invention exhibits an increasing symptom of the protein of the present invention liberated, the animal is usable for screening test of preventive/therapeutic agents for diseases associated with the protein of the present invention, for example, the preventive/therapeutic agent for arteriosclerotic disease, diabetic complications, etc.

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the animal after DNA transfection means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention is expressed to a high level, and may eventually develop the function inactive type inadaptability to the protein of the present invention by inhibiting the functions of endogenous normal DNA. Therefore, the animal can be utilized as a pathologic model animal for such a disease. For example, using the abnormal DNA transgenic animal of the present invention, it is possible to elucidate the mechanism of the function inactive type inadaptability to the protein of the present invention and the pathological mechanism of the disease associated with the protein of the present invention and to investigate how to treat the disease.

More specifically, the transgenic animal of the present invention expressing the abnormal DNA of the present invention at a high level is expected to serve as an experimental model to elucidate the mechanism of the functional inhibition (dominant negative effect) of a normal protein by the abnormal protein of the present invention in the function inactive type inadaptability of the protein of the present invention.

Since a mammal bearing the abnormal exogenous DNA of the present invention shows an increased symptom of the protein of the present invention liberated, the animal is also expected to serve for screening test of the protein of the present invention or the preventive/therapeutic agents for the function inactive type inadaptability.

Other potential applications of two kinds of the DNA transgenic animals of the present invention described above further include, for example:

1) use as a cell source for tissue culture;
2) elucidation of the relation to a peptide that is specifically expressed or activated by the protein of the present invention, by direct analysis of DNA or RNA in tissues of the DNA transgenic animal of the present invention or by analysis of the peptide tissues expressed by the DNA;
3) research on the function of cells derived from tissues that are usually cultured only with difficulty, using cells in tissues bearing the DNA cultured by a standard tissue culture technique;
4) screening a drug that enhances the functions of cells using the cells described in (iii) above; and,
5) isolation and purification of the variant protein of the present invention and preparation of an antibody thereto; etc.

Furthermore, clinical conditions of a disease associated wit the protein of the present invention, including the function inactive type inadaptability to the protein of the present invention can be determined by using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve to identify cells capable of producing the protein of the present invention, and to study in association with apoptosis, differentiation or propagation or on the mechanism of signal transduction in these properties to inspect any abnormality therein. Thus, the DNA transgenic animal can provide an effective research material for the protein of the present invention and for investigation of the function and effect thereof.

To develop a medicament for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

**(8) Knockout animal**

The present invention provides a non-human mammal embryonic stem cell bearing the DNA of the present invention inactivated and a non-human mammal deficient in expressing the DNA of the present invention.

Thus, the present invention provides:

(1) a non-human mammal embryonic stem cell in which the DNA of the present invention is inactivated;
(2) the embryonic stem cell according to (1), wherein the DNA is inactivated by introducing a reporter gene (e.g., β-galactosidase gene derived from *Escherichia coli*);
(3) the embryonic stem cell according to (1), which is resistant to neomycin;
(4) the embryonic stem cell according to (1), wherein the non-human mammal is a rodent;
(5) the embryonic stem cell according to (4), wherein the rodent is mouse;
(6) a non-human mammal deficient in expressing the DNA of the present invention, wherein the DNA is inactivated;
(7) the non-human mammal according to (6), wherein the DNA is inactivated by inserting a reporter gene (e.g., β-galactosidase derived from *Escherichia coli*) therein and the reporter gene is capable of being expressed under control of a promoter for the DNA of the present invention;
(8) the non-human mammal according to (6), which is a rodent;
(9) the non-human mammal according to (8), wherein the rodent is mouse; and,
(10) a method of screening a compound or its salt that promotes or inhibits the promoter activity to the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).

As the non-human mammal, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter simply referred to as a targeting vector).

The thus-obtained ES cells to the southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the $BDF_1$ mouse ($F_1$ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The $BDF_1$ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about $10^6$ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1% trypsin/1 mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for studying the function of the protein of the present invention cytologically.

The non-human mammal deficient in expression of the DNA of the present invention can be identified from a normal animal by measuring the mRNA level in the subject animal by a publicly known method, and indirectly comparing the degrees of expression.

As the non-human mammal, the same examples given above apply.

With respect to the non-human mammal deficient in expression of the DNA of the present invention, the DNA of the present invention can be knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned;

the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygous animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal deficient in expression of the DNA of the present invention lacks various biological activities derived from the protein of the present invention, such an animal can be a disease model suspected of inactivated biological activities of the protein of the present invention and thus, offers an effective study to investigate the causes for and therapy for these diseases.

**(8a) Method of screening compounds having therapeutic/preventive effects on disease caused by deficiency, damages, etc. of DNA of the present invention**

The non-human mammal deficient in expression of the DNA of the present invention can be employed for screening the compound having a therapeutic/prophylactic effect on diseases caused by deficiency, damages, etc. of the DNA of the present invention.

That is, the present invention provides a method of screening the compound or its salt having therapeutic/ preventive effect on diseases caused by deficiency, damages, etc. of the DNA of the present invention, which comprises administering a test compound to a non-human mammal deficient in expression of the DNA of the present invention and, observing and measuring changes occurred in the animal.

As the non-human mammal deficient in expression of the DNA of the present invention, which can be employed for the screening method, the same examples as described above apply.

Examples of the test compound include peptides, proteins, antibodies, non-peptide compounds, synthetic compounds, fermentation products, cell extracts, plant extracts, animal tissue extracts, blood plasma, etc. These compounds may be novel compounds or publicly known compounds. The test compound may form salts, and these salts of the test compound used are the same as those given above.

Specifically, the non-human mammal deficient in expression of the DNA of the present invention is treated with a test compound, comparison is made with an intact animal for control and changes in each organ, tissue, disease conditions, etc. of the animal is used as an indicator to assess therapeutic/preventive effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately chosen depending on the administration route, nature of the test compound, etc.

For screening of the compound having therapeutic/preventive effects on, e.g., arteriosclerotic disease, diabetic complications, etc., a test compound is administered to the non-human mammal deficient in expression of the DNA of the present invention. Differences in incidence of arteriosclerotic disease or differences in degree of healing from arteriosclerotic disease in the group administered with no test compound are observed in the tissues described above with passage of time.

In the screening method, when a test compound is administered to a test animal and the disease conditions of the test animal are improved by at least about 10%, preferably at least about 30% and more preferably at least about 50%, the test compound can be selected as the compound having therapeutic/preventive effect on the diseases described above.

The compound obtained using the above screening method is a compound selected from the test compounds described above and exhibits therapeutic/preventive effects on diseases caused by enhanced expression, etc. of the protein of the present invention. Therefore, the compound can be employed as a safe and low toxic drug for the prevention/ treatment of these diseases. Furthermore, compounds derived from the compound obtained by the screening described above may also be used as well. The diseases caused by the enhanced expression, etc. of the protein of the present invention include, for example, arteriosclerotic disease, diabetic complications, etc. Accordingly, the compound or its salt that inhibits the activity of the protein of the present invention, the compound or its salt that inhibits the expression of a gene for the protein of the present invention, and the compound or its salt that inhibits the production of the protein of the present invention, etc. can be used as preventive/therapeutic agents for diseases such as arteriosclerotic disease, diabetic complications, etc.

The compound obtained by the screening method may form salts, and the salts for the compound used are the same as those given for the test compound described above.

The medicament comprising the compound or its salt, which is obtained by the above screening method, can be prepared in a manner similar to the method for preparing the medicament comprising the antibody to the protein of the present invention described hereinabove.

Since the pharmaceutical preparation thus obtained is safe and low toxic, it can be administered to human or a mammal (e.g., rat, mouse, guinea pig, rabbit, sheep, swine, bovine, horse, cat, dog, monkey, etc.).

The dose of the compound or its salt may vary depending upon target disease, subject to be administered, conditions, route of administration, etc. For example, when the compound or its salt is orally administered for the treatment of arteriosclerotic disease (e.g., myocardial infarction, unstable angina, etc.), the compound or its salt is administered to an adult (as 60 kg body weight) generally in a daily dose of about 0.1 to 100 mg, preferably about 1.0 to 50 mg and more preferably about 1.0 to 20 mg. In parenteral administration, a single dose of the compound may vary depending upon target disease, subject to be administered, conditions, route for administration, etc. When the compound or its salt is administered for the treatment of arteriosclerotic disease (e.g., myocardial infarction, unstable angina, etc.), it is advantageous to administer the compound or its salt to an adult (as 60 kg body weight) in the form of an injectable preparation, in a single dose of about 0.01 to about 30 mg, preferably about 0.1 to about 20 mg and more preferably about 0.1 to about 10 mg per day. For other animal species, the corresponding dose as converted per 60 kg weight can be administered.

**(8b) Method of screening compounds that inhibit the activity of promoter to DNA of the present invention**

The present invention provides a method of screening a compound or its salt that promotes or inhibits the activity of a promoter to the DNA of the present invention, which comprises administering a test compound to a non-human mammal deficient in expression of the DNA of the present invention and detecting the expression of a reporter gene.

In the screening method described above, an animal in which the DNA of the present invention is inactivated by introducing a reporter gene and the reporter gene can be expressed under control of a promoter to the DNA of the present invention is used as the non-human mammal deficient in expression of the DNA of the present invention, which is selected from the aforesaid non-human mammals deficient in expression of the DNA of the present invention.

The same examples of the test compound apply to specific compounds described above. The test compound may form salts, and these salts of the test compound used are the same as those given above.

As the reporter gene, the same specific examples apply to this screening method. Preferably, there are used β-galactosidase (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from Escherichia coli, β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or its salt obtained using the screening method described above are compounds that are selected from the test compounds described above and that promote or inhibit the promoter activity to the DNA of the present invention.

The compound obtained by the screening method may form salts, and these salts of the compound used are the same as those given for the test compound described above.

The compound or its salt that regulates (promotes or inhibits, preferably inhibits) the promoter activity to the DNA of the present invention can regulate (promote or inhibit, preferably inhibit) the expression of the protein of the present invention to regulate (promote or inhibit, preferably inhibit) the functions of the protein. Thus, the compound or its salt is useful as preventive/therapeutic agents for, e.g., arteriosclerotic disease, diabetic complications, etc.

In addition, compounds derived from the compound obtained by the screening described above may also be used as well.

The medicament comprising the compound or its salt obtained by the above screening method can be prepared in a manner similar to the method for preparing the medicament comprising the antibody to the protein of the present invention described above.

Since the pharmaceutical preparation thus obtained is safe and low toxic, it can be administered to human or a mammal (e.g., rat, mouse, guinea pig, rabbit, sheep, swine, bovine, horse, cat, dog, monkey, etc.).

A dose of the compound or its salt may vary depending on target disease, subject to be administered, conditions, route for administration, etc.; when the compound or its salt that regulates (promotes or inhibits; preferably inhibits) the promoter activity to the DNA of the present invention is orally administered for the treatment of arteriosclerotic disease (e.g., myocardial infarction, unstable angina, etc.), the compound is administered to an adult (as 60 kg body weight) normally in a daily dose of about 0.1 to 100 mg, preferably about 1.0 to 50 mg and more preferably about 1.0 to 20 mg. In parenteral administration, a dose of the compound may vary depending on target disease, subject to be administered, conditions, route for administration, etc. but when the compound or its salt that regulates (promotes or inhibits; preferably inhibits) the promoter activity to the DNA of the present invention is administered to an adult (as 60 kg body weight) in the form of injectable preparation, it is advantageous to administer the compound intravenously in a daily dose of about 0.01 to about 30 mg, preferably about 0.1 to about 20 mg and more preferably about 0.1 to about 10 mg. For other animal species, the corresponding dose as converted per 60 kg weight can be administered.

As stated above, the non-human mammal deficient in expression of the DNA of the present invention is extremely useful for screening the compound or its salt that promotes or inhibits the promoter activity to the DNA of the present invention and, can greatly contribute to elucidation of causes for various diseases suspected of deficiency in expression of the DNA of the present invention and for the development of preventive/therapeutic agents for these diseases.

In addition, a so-called transgenic animal (gene transferred animal) can be prepared by using a DNA containing the promoter region of the protein of the present invention, ligating genes encoding various proteins at the downstream and injecting the same into oocyte of an animal. It is thus possible to synthesize the protein therein specifically and study its activity in vivo. When an appropriate reporter gene is ligated to the promoter site described above and a cell line that expresses the gene is established, the resulting system can be utilized as the search system for a low molecular compound having the action of specifically promoting or suppressing the in vivo productivity of the protein itself of the present invention.

In the specification, when bases, amino acids, etc. are denoted by abbreviations, these codes are in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA | : deoxyribonucleic acid |
| cDNA | : complementary deoxyribonucleic acid |
| A | : adenine |
| T | : thymine |
| G | : guanine |
| C | : cytosine |
| RNA | : ribonucleic acid |
| mRNA | : messenger ribonucleic acid |
| dATP | deoxyadenosine triphosphate |
| dTTP | : deoxythymidine triphosphate |
| dGTP | : deoxyguanosine triphosphate |
| dCTP | : deoxycytidine triphosphate |
| ATP | : adenosine triphosphate |

EDTA ethylenediaminetetraacetic acid
SDS : sodium dodecyl sulfate
Gly : glycine
Ala : alanine
Val : valine
Leu : leucine
Ile : isoleucine
Ser : serine
Thr : threonine
Cys : cysteine
Met : methionine
Glu : glutamic acid
Asp : aspartic acid
Lys : lysine
Arg : arginine
His : histidine
Phe : phenylalanine
Tyr : tyrosine
Trp : tryptophan
Pro : proline
Asn : asparagine
Gln : glutamine
pGlu : pyroglutamic acid
Sec : selenocysteine

Substituents, protecting groups and reagents generally used in this specification are presented as the codes below.

Me : methyl group
Et : ethyl group
Bu : butyl group
Ph : phenyl group
TC : thiazolidine-4(R)-carboxamido group
Tos : p-toluenesulfonyl
CHO :formyl
Bzl : benzyl
$Cl_2$-Bzl : 2,6-dichlorobenzyl
Bom : benzyloxymethyl
Z : benzyloxycarbonyl
Cl-Z :2-chlorobenzyloxycarbonyl
Br-Z :2-bromobenzyloxycarbonyl
Boc : t-butoxycarbonyl
DNP : dinitrophenol
Trt : trityl
Bum : t-butoxymethyl
Fmoc : N-9-fluorenylmethoxycarbonyl
HOBt : 1-hydroxybenztriazole
HOOBt : 3,4-dihydro-3-hydroxy-4-oxo-1,2,3-benzotriazine
HONB :1-hydroxy-5-norbornene-2,3-dicarboxyimide
DCC : N,N'-dicyclohexylcarbodiimide

The sequence identification numbers in the sequence listing of the specification indicate the following sequences.

[SEQ ID NO: 1]
This shows the amino acid sequence of glucosylceramide synthase.
[SEQ ID NO: 2]
This shows the base sequence of DNA encoding glucosylceramide synthase having the amino acid sequence represented by SEQ ID NO: 1.
[SEQ ID NO: 3]
This shows the amino acid sequence of lactosylceramide synthase.
[SEQ ID NO: 4]
This shows the base sequence of DNA encoding lactosylceramide synthase having the amino acid sequence represented by SEQ ID NO: 3.
[SEQ ID NO: 5]
This shows the base sequence of sense primer 1 used in EXAMPLE 1.
[SEQ ID NO: 6]
This shows the base sequence of antisense primer 2 used in EXAMPLE 1.
[SEQ ID NO: 7]
This shows the base sequence of sense primer 3 used in EXAMPLE 1.
[SEQ ID NO: 8]
This shows the base sequence of antisense primer 4 used in EXAMPLE 1.
[SEQ ID NO: 9]
This shows the base sequence of primer used in EXAMPLE 4.
[SEQ ID NO: 10]
This shows the base sequence of the primer used in EXAMPLE 4.
[SEQ ID NO: 11]
This shows the amino acid sequence of GM3 synthase.
[SEQ ID NO: 12]
This shows the base sequence of DNA encoding GM3 synthase having the amino acid sequence represented by SEQ ID NO: 11.

## EXAMPLES

Hereinafter the present invention will be described more specifically with reference to EXAMPLES, but is not deemed to be limited.

### EXAMPLE 1

Study on the expression of glucosylceramide synthase and lactosylceramide synthase in arteriosclerotic lesions of rabbit aorta

New Zealand White rabbit (Kbl:NZW) of 5 weeks old was fed a 0.5% cholesterol-contained RC-4 diet for 8 weeks and then RC-4 diet for further 4 weeks. After blood was exsanguinated from the carotid artery under anesthesia, sampling was conducted from the aortic root to the thoracic aorta. The fat and connective tissues, etc. were trimmed from the aorta in chilled physiological saline and stored in chilled RNAlater (manufactured by Qiagen). The aorta was visually bifurcated into two parts, more (severe) and less arteriosclerotic lesions. Total RNA was prepared using RNeasy Fibrous Tissue Midi Kit (manufactured by Qiagen), and cDNA was prepared using TaqMan Reverse Transcription Reagents (manufactured by Applied Biosystems). PCR was carried out using TaqMan Universal PCR Master Mix (manufactured by Applied Biosystems), followed by agarose electrophoresis to comparatively evaluate expression levels semiquantitatively. PCR primer was designed based on base sequence information of partial sequences of rabbit glucosylceramide synthase and lactosylceramide synthase acquired.

Using sense primer 1 (SEQ ID NO: 5) and antisense primer 2 (SEQ ID NO: 6) of glucosylceramide synthase, PCR was carried out under the conditions at an annealing temperature of 54°C with the cycle number of 36.

Using sense primer 3 (SEQ ID NO: 7) and antisense primer 4 (SEQ ID NO: 8) of lactosylceramide synthase, PCR was carried out under conditions at an annealing temperature of 55°C with the cycle number of 34.

As a result of agarose gel electrophoresis, comparison between the same subject showed that mRNA expression of glucosylceramide synthase and lactosylceramide synthase was more enhanced in the vascular part with a more (severe) arteriosclerotic lesion, as compared to the vascular part with obviously a less arteriosclerotic lesion. Accordingly, the activities of both synthases would be shown to be elevated.

EXAMPLE 2

(1) Effects of D-PDMP

(D-threo-1-phenyl-2-decanoylamino-3-morpholino-1-propanol) on cholesterol efflux activity in THP-1 cells

D-PDMP is known to inhibit the activities of glucosylceramide synthase and lactosylceramide synthase (Glycoconjugate J., 13, 481-486, 1996), and is known to be able to deplete glucosylceramide and lactosylceramide in cells in a concentration- and treating time-dependent manner (J. Biochem. (Tokyo), 117, 766-773, 1995). Using this compound, effects on the cholesterol efflux activity was assessed when accumulation of glycolipids in the cells was inhibited.

THP-1 cells (purchased fromATCC; Int. J. Cancer., 26, 171-176, 1980) seeded in a 12-well plate (Falcon) at 8.0 x $10^5$/well were treated for 3 days in RPMI-1640 medium (supplemented with 10% FBS, 25 mM HEPES and penicillin/streptomycin) containing phorbol-12-myristate-13-acetate (PMA) (final concentration: 100 nM) to allow for differentiation into macrophage-like cells.

After washing with PBS, serum-free RPMI-1640 containing [$^3$H] cholesterol-labeled βVLDL (final cholesterol level: 150 μg/ml) was added thereto and incubated for 24 hours to prepare foamed cells. After washing twice with 0.2 % BSA-containing PBS and then once with PBS, serum-free RPMI-1640 media supplemented with 0, 10 and 20 μM D-PDMP, respectively, were added to treat for 24 and 48 hours. The supernatant was removed and RPMI-1640 medium (final concentration: 25 μg/ml) containing human apolipoprotein AI (Chemicon) was added thereto. Six hours after, the radioactivity released into the supernatant was measured in a liquid scintillation counter. The cells were further washed once with PBS and then lysed in 0.1% SDS-containing 0.1 N NaOH aqueous solution. A part of the lysate was taken up and the radioactivity was measured in a liquid scintillation counter.

The activity in efflux of cholesterol was determined by the following equation:

$$\text{Cholesterol efflux activity (\%)} = \text{Radioactivity in supernatant}/(\text{Radioactivity in supernatant} + \text{Radioactivity in cell lysate}) \times 100$$

The results are shown in TABLE 1. Numerical values are presented in mean ± standard deviation.

[TABLE 1]

| | Cholesterol Efflux Activity (%) | |
|---|---|---|
| D-PDMP (μM) | 24 hrs. | 48 hrs. |
| 0 | 1.7±0.3 | 2.1±0.2 |
| 10 | 4.4±0.4 | 6.0±0.4 |
| 20 | 4.3±0.8 | 6.8±0.5 |

As shown in TABLE 1, D-PDMP increased the cholesterol efflux activity concentration- and time-dependently. This indicates that inhibition of the glucosylceramide synthase activity and lactosylceramide synthase activity promotes the cholesterol efflux activity in cells to cause regression of arteriosclerosis.

(2) Effects of sphingoglycolipid on cholesterol efflux activity in THP-1 cells

Cells were prepared as described in (1) above. After the foamed cells prepared were washed, serum-free RPMI-1640 media containing 10 μM of glucosylceramide (Matreya, Inc.) and lactosylceramide (CALBIOCHEM), respectively, were added to treat for 24 hours. The supernatant was removed and RPMI-1640 medium (final concentration: 25 μg/ml) containing human apolipoprotein AI (Chemicon) was added thereto. Six hours after, the cholesterol efflux activity was measured by the same method as described in (1) above.

The results are shown in TABLES 2 and 3. The numerical values are presented in mean ± standard deviation.

[TABLE 2]

| Glucosylceramide (μM) | Cholesterol Efflux Activity (%) |
|---|---|
| 0 | 3.6±0.1 |
| 10 | 3.2±0.2 |

[TABLE 3]

| Lactosylceramide (μM) | Cholesterol Efflux Activity (%) |
|---|---|
| 0 | 4.3±0.2 |
| 10 | 2.3±0.2 |

As shown in TABLES 2 and 3, the cholesterol efflux activity was suppressed by exogenously adding glucosylceramide or lactosylceramide to the culture system. The foregoing results show that intracellular accumulation of glucosylceramide and lactosylceramide acts to promote the formation of arteriosclerosis. This establishes that such compounds that reduce intracellular glycosphingolipids, e.g., compounds that inhibit the glucosylceramide synthase activity or (and) lactosylceramide synthase activity are effective as medicaments for suppressing the development of arteriosclerosis.

EXAMPLE 3

Effects of D-PDMP (D-threo-1-phenyl-2-decanoylamino-3-morpholino-1-propanol) on denatured lipoprotein-uptake activity in THP-1 cells

THP-1 cells (purchased from ATCC) seeded in a 6-well Low Cell Binding Plate (Nunc) at 1.5 x $10^6$/well were treated for 2 days in RPMI-1640 medium (supplemented with 0.4% FBS, 25 mM HEPES and penicillin/streptomycin) containing phorbol-12-myristate-13-acetate (PMA) (final concentration: 100 nM) or in RPMI-1640 medium (supplemented with 0.4% FBS, 25 mM HEPES and penicillin/streptomycin) containing PMA (final concentration: 100 nM) and D-PDMP (final concentration: 20 μM). Next, the treated cells were washed with serum-free RPMI-1640 medium and then suspended in serum-free RPMI-1640 medium supplemented with DiI (1,1'-dioctadecyl-3,3,3',3'-tetramethylindocarbocyanine)-labeled acetyl LDL (Funakoshi, final concentration: 20 μg/ml), followed by incubation at 37°C for 90 minutes while shaking. After washing with PBS, the cells were suspended in Cellfix buffer (BD) and uptake of DiI-labeled acetyl LDL was analyzed on a FACScan (BD Bioscience). The intermediate value obtained by the FACS analysis of the cells treated with phorbol-12-myristate-13-acetate alone was made 100%, and relative values were determined under each condition.
The results are shown in TABLE 4. The numerical values are presented in mean ± standard deviation.

[TABLE 4]

| | Acetyl LDL Uptake Activity (%) |
|---|---|
| Addition of PMA | 100±18.2 |
| Addition of PMA and D-PDMP | 28.9±6.3 |

It has become clear from TABLE 4 that D-PDMP suppressed the acetyl LDL uptake activity.
The results above reveal that the uptake of denatured lipoproteins into the cells was suppressed in macrophages by inhibiting the glucosylceramide synthase activity and the lactosylceramide synthase activity. In other words, the results indicate that the compounds inhibiting the glucosylceramide synthase activity or (and) the lactosylceramide synthase activity are effective as anti-arteriosclerotic drugs by inhibition of foam cell formation based on suppression of the uptake of denatured lipoproteins in monocytes or macrophages infiltrated in arteriosclerotic lesions.

EXAMPLE 4

(1) Construction of glucosylceramide synthase expression vector

Double stranded DNA having the base sequence represented by SEQ ID NO: 2 was synthesized by PCR using two primers (SEQ ID NO: 9 and SEQ ID NO: 10).
Human glucosylceramide synthase gene was obtained from IMAGE clone 5274768 (Invitrogen). The reaction solution for the reaction was composed of 2 μl of plasmid DNA of IMAGE clone 5274768, 5 μl of 10 x Pfu buffer (Stratagene), 1 μl of Hotstart Pfu (Stratagene), 1 μl each of 20 μM primers and 4 μl of 2.5 mM dNTP, to which 36 μl of distilled water was added to make the volume 50 μl After 2 minutes at 95°C, PCR was carried out by repeating 30 cycles, one set to include 95 °C for 30 seconds, 60 °C for 30 seconds and 72 °C for 2 minutes, followed by an extension at 72°C for 10 minutes. After completion of the reaction, 500 ng of the PCR product, which was purified using PCR purification kit (Qiagen), was digested with restriction enzymes NotI and BamHI, followed by purification with Cleanup kit (Qiagen). In a similar way pcDNA3.1(-) vector (Invitrogen) was digested with restriction enzymes NotI and BamHI, followed by purification with Cleanup kit (Qiagen). The PCR product, 100 ng, and 50 ng of pcDNA3.1(-) vector fragment were mixed with 5 μl of DNA ligation kit Ver. 2 Solution I, followed by ligation at 16°C for 30 minutes. After the ligation reaction, the ligation product was transformed in Escherichia coli DH5α. The resulting transformant was cultured, and then plasmid DNA was extracted. Using Big Dye Terminator cycle sequencing ver. 3.1, sequencing was performed to identify the base sequence with 3100 DNA sequencer, and a clone identical with the base sequence represented by SEQ ID NO: 2 (encompassing all ORFs) was selected.

(2) Screening of glucosylceramide synthase inhibitor

The enzyme source was prepared by the procedure shown below.

HEK293 cells (purchased from ATCC; J. Gen. Virol., 36, 59-74, 1977) were cultured in serum-containing DMEM medium and seeded in a 10 cm collagen coated plate (Iwaki Co.) at 1.2 x $10^6$ on the previous day of gene transfer. After washing with serum-free DMEM medium, 4 μg of the human glucosylceramide synthase expression vector obtained in (1) above, 30 μl of Lipofectamine (Invitrogen) and 20 μl of PLUS Reagent (Invitrogen) were added to 1.5 ml of OPTI MEM-I medium (Invitrogen) to carry out gene transfer. After culturing under conditions at 37°C in 5% $CO_2$ and washing with PBS, the cells were lysed using 1 ml of M-PER (Pierce), followed by centrifugation at 27,000 x g for 10 minutes. The resulting soluble fraction was used as the enzyme source. For the enzyme assay, 10 μg of crude enzyme solution, 500 nmol of Tris buffer (pH 7.5), 30 nmol of C8 ceramide (BIOMOL) and 375 pmol of [$^{14}$C] UDPglucose (Amersham, 7.4 GBq/mmol, 925 Kbq/ml) were incubated in 50 μl of the reaction solution at 32°C for 2 hours. After 100 μl of chloroform-methanol (2:1) was added to terminate the reaction, centrifugation was performed at 15000 rpm for 5 minutes. Then, the chloroform layer was collected and the radioactivity was measured in a liquid scintillation counter. The activity value was determined by the following equation:

$$\text{Glucosylceramide synthase activity (\%)} = \text{Radioactivity of the enzyme reaction product (cpm)/Specific radioactivity of } [^{14}\text{C}]\text{UDP-glucose (cpm/nmol)} \times 1/\text{amount of protein (mg)} \cdot \text{reaction time (hr)}$$

The results measured are shown in TABLE 5. The numerical values are presented in mean ± standard deviation.

[TABLE 5]

| | Glucosylceramide synthase activity (nmol/mg/hr) |
|---|---|
| Enzyme: none | 0.02±0.015 |
| Enzyme: present | 1.90±0.224 |

D-PDMP known to inhibit the glucosylceramide synthase activity was examined using the procedure described above. The $IC_{50}$ was calculated using SAS Preclinical Package, ver. 5 and found to be 1.5 μM. By using this enzyme assay system, screening of the glucosylceramide synthase is performed.

INDUSTRIAL APPLICABILITY

The protein used in the present invention is overexpressed specifically in arteriosclerotic lesions. Thus, the compound or its salt that inhibits the activity of glucosylceramide synthase or (and) lactosylceramide synthase, the compound or its salt that inhibits the expression of glucosylceramide synthase gene or (and) lactosylceramide synthase gene, the compound or its salt that inhibits the production of glucosylceramide synthase or (and) lactosylceramide synthase, etc. can be safely used as agents for preventing/treating, for example, arteriosclerosis or arteriosclerotic disease (e.g., cerebral artery disease (e.g., cerebral infarction, cerebral hemorrhage, etc.); coronary artery disease (e.g.,

ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.), and so on.

Furthermore, (a) the compound or its salt (i) that inhibits the activity of glucosylceramide synthase or (and) lactosylceramide synthase and (ii) that inhibits the activity of GM3 synthase, (b) the compound or its salt (i) that inhibits the expression of glucosylceramide synthase gene or (and) lactosylceramide synthase gene and (ii) that inhibits the expression of GM3 synthase gene, (c) the compound or its salt (i) that inhibits the production of glucosylceramide synthase or (and) lactosylceramide synthase and (ii) that inhibits the production of GM3 synthase, etc. can be safely used as agents for preventing/treating, for example, arteriosclerosis or arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.), and so on.

Moreover, the glucosylceramide synthase or (and) lactosylceramide synthase is useful for screening an agent for preventing/treating arteriosclerosis or arteriosclerotic disease (e.g., cerebral artery disease (e.g., cerebral infarction, cerebral hemorrhage, etc.); coronary artery disease (e.g., ischemic heart disease such as myocardial infarction, angina pectoris, etc.); aortic disease (e.g., aortic aneurysm, aortic dissection, etc.); renal artery disease (e.g., nephrosclerosis, renal failure caused by nephrosclerosis, etc.); peripheral arterial disease (e.g., arteriosclerosis obliterans, etc.), etc.); diabetes mellitus or diabetic complications (e.g., diabetic retinopathy, diabetic neuropathy, diabetic nephropathy, etc.); metabolic syndrome (syndrome implicating multiple risk factors such as insulin resistance-mediated hyperglycemia, hyperlipemia, hypertension, etc.), and so on.

## Claims

1. A medicament for preventing/treating arteriosclerosis, which comprises a glucosylceramide synthase inhibitor or (and) a lactosylceramide synthase inhibitor.

2. A medicament for preventing/treating arteriosclerosis, which comprises an expression inhibitor of glucosylceramide synthase gene or (and) an expression inhibitor of lactosylceramide synthase gene.

3. The medicament according to claim 1 or 2, wherein the glucosylceramide synthase is a protein comprising the same or substantially the same amino acid sequence as the amino acid sequence represented by SEQ ID NO: 1, its partial peptide, or a salt thereof.

4. The medicament according to claim 1 or 2, wherein the glucosylceramide synthase is a protein consisting of the amino acid sequence represented by SEQ ID NO: 1, or a salt thereof.

5. The medicament according to claim 1 or 2, wherein the lactosylceramide synthase is a protein comprising the same or substantially the same amino acid sequence as the amino acid sequence represented by SEQ ID NO: 3, its partial peptide, or a salt thereof.

6. The medicament according to claim 1 or 2, wherein the lactosylceramide synthase is a protein consisting of the amino acid sequence represented by SEQ ID NO: 3, or a salt thereof.

7. The medicament according to claim 1, which further comprises a GM3 synthase inhibitor.

8. The medicament according to claim 2, which further comprises an expression inhibitor of GM3 synthase gene.

9. The medicament according to claim 7 or 8, wherein the GM3 synthase is a protein comprising the same or substantially the same amino acid sequence as the amino acid sequence represented by SEQ ID NO: 11, its partial peptide, or a salt thereof.

10. The medicament according to claim 7 or 8, wherein the GM3 synthase is a protein consisting of the amino acid sequence represented by SEQ ID NO: 11, or a salt thereof.

11. An antisense polynucleotide, which comprises the entire or part of base sequence complementary or substantially complementary to a base sequence of a polynucleotide encoding a protein comprising the same or substantially the same amino acid sequence represented by SEQ ID NO: 1 or SEQ ID NO: 3, or a partial peptide thereof.

12. A medicament, which comprises an antisense polynucleotide comprising the entire or part of base sequence complementary or substantially complementary to a base sequence of a polynucleotide encoding a protein comprising the same or substantially the same amino acid sequence represented by SEQ ID NO: 1 or (and) SEQ ID NO: 3, or a partial peptide thereof.

13. The medicament according to claim 12, which is a medicament for preventing/treating arteriosclerosis.

14. The medicament according to claim 13, which further comprises an antisense polynucleotide comprising the entire or part of base sequence complementary or substantially complementary to a base sequence of a polynucleotide encoding a protein comprising the same or substantially the same amino acid sequence as the amino acid sequence represented by SEQ ID NO: 11, or a partial peptide thereof.

15. A siRNA or shRNA for a polynucleotide encoding a protein comprising the same or substantially the same amino acid sequence represented by SEQ ID NO: 1 or SEQ ID NO: 3, or a partial peptide thereof.

**16.** A medicament, which comprises a siRNA or shRNA for a polynucleotide encoding a protein comprising the same or substantially the same amino acid sequence represented by SEQ ID NO: 1 or (and) SEQ ID NO: 3, or a partial peptide thereof.

**17.** The medicament according to claim 16, which is a medicament for preventing/treating arteriosclerosis.

**18.** The medicament according to claim 17, which further comprises a siRNA or shRNA for a polynucleotide encoding a protein comprising the same or substantially the same amino acid sequence as the amino acid sequence represented by SEQ ID NO: 11, or a partial peptide thereof.

**19.** A medicament for preventing/treating arteriosclerosis, which comprises an antibody to a glucosylceramide synthase or (and) an antibody to a lactosylceramide synthase.

**20.** The medicament according to claim 19, which further comprises an antibody to a GM3 synthase.

**21.** A diagnostic agent for arteriosclerosis, which comprises an antibody to a glucosylceramide synthase or (and) an antibody to a lactosylceramide synthase.

**22.** The diagnostic agent according to claim 21, which further comprises an antibody to a GM3 synthase.

**23.** A method for diagnosis of arteriosclerosis, which comprises using an antibody to a glucosylceramide synthase or (and) an antibody to a lactosylceramide synthase.

**24.** The method for diagnosis according to claim 23, which further comprises using an antibody to a GM3 synthase.

**25.** A diagnostic agent for arteriosclerosis, which comprises a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

**26.** The diagnostic agent according to claim 25, which further comprises a polynucleotide encoding a protein comprising the same or substantially the same amino acid sequence as the amino acid sequence represented by SEQ ID NO: 11, or a partial peptide thereof.

**27.** A method for diagnosis of arteriosclerosis, which comprises using a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

**28.** The method for diagnosis according to claim 27, which further comprises using a polynucleotide encoding a protein comprising the same or substantially the same amino acid sequence as the amino acid sequence represented by SEQ ID NO: 11, or a partial peptide thereof.

**29.** Use of an antibody to a glucosylceramide synthase or (and) an antibody to a lactosylceramide synthase to manufacture a diagnostic agent for arteriosclerosis.

**30.** Use of a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof, to manufacture a diagnostic agent for arteriosclerosis.

**31.** A method for diagnosis of arteriosclerosis, which comprises assaying the level of a glucosylceramide or (and) a lactosylceramide in plasma of a mammal.

**32.** The method for diagnosis according to claim 31, which further comprises assaying the level of GM3.

**33.** Use of a glucosylceramide or (and) a lactosylceramide as a diagnostic marker for arteriosclerosis.

**34.** The use according to claim 33, which further comprises use of GM3.

**35.** A method of screening an agent for preventing/treating arteriosclerosis, which comprises using a protein comprising the same or substantially the same amino acid sequence as the amino acid sequence represented by SEQ ID NO: 1, its partial peptide, or a salt thereof, or (and) a protein comprising the same or substantially the same amino acid sequence as the amino acid sequence represented by SEQ ID NO: 3, its partial peptide, or a salt thereof.

**36.** The screening method according to claim 35, which further comprises using a protein comprising the same or substantially the same amino acid sequence as the amino acid sequence represented by SEQ ID NO: 11, its partial peptide, or a salt thereof.

**37.** A kit for screening an agent for preventing/treating arteriosclerosis, which comprises a protein comprising the same or substantially the same amino acid sequence as the amino acid sequence represented by SEQ ID NO: 1, its partial peptide, or a salt thereof or (and) a protein comprising the same or substantially the same amino acid sequence as the amino acid sequence represented by SEQ ID NO: 3, its partial peptide, or a salt thereof.

**38.** The screening kit according to claim 37, which further comprises a protein comprising the same or substantially the same amino acid sequence as the amino acid sequence represented by SEQ ID NO: 11, its partial peptide, or a salt thereof.

**39.** A method of screening an agent for preventing/treating arteriosclerosis, which comprises using a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

**40.** A method of screening an agent for preventing/treating arteriosclerosis, which comprises using a polynucleotide encoding a protein comprising the same or substantially the same amino acid sequence as the amino acid sequence represented by SEQ ID NO: 11, or a partial peptide thereof.

**41.** A kit for screening an agent for preventing/treating arteriosclerosis, which comprises a polynucleotide encoding a protein comprising the same or substantially the same amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

**42.** The screening kit according to claim 41, which further comprises a polynucleotide encoding a protein comprising the same or substantially the same amino acid sequence as the amino acid sequence represented by SEQ ID NO: 11, or a partial peptide thereof.

**43.** A method of screening an agent for preventing/treating arteriosclerosis, which comprises assaying the activity or level of a protein comprising the same or substantially the same amino acid sequence represented by SEQ ID NO: 1, its partial peptide, or a salt thereof, or (and) a protein comprising the same or substantially the same amino acid sequence as the amino acid sequence represented by SEQ ID NO: 3, its partial peptide, or a salt thereof.

**44.** The screening method according to claim 43, which further comprises assaying the activity or level of a protein comprising the same or substantially the same amino acid sequence represented by SEQ ID NO: 11, its partial peptide, or a salt thereof.

**45.** A method of screening an agent for preventing/treating arteriosclerosis, which comprises assaying the level of a polynucleotide encoding a protein comprising the same or substantially the same amino acid sequence as the amino acid sequence represented by SEQ ID NO: 1, or a partial peptide thereof, or (and) a polynucleotide encoding a protein comprising the same or substantially the same amino acid sequence as the amino acid sequence represented by SEQ ID NO: 3, or a partial peptide thereof.

**46.** The screening method according to claim 45, which further comprises assaying the level of a polynucleotide encoding a protein comprising the same or substantially the same amino acid sequence as the amino acid sequence represented by SEQ ID NO: 11, or a partial peptide thereof.

**47.** A method of preventing/treating arteriosclerosis, which comprises inhibiting the activity of a glucosylceramide synthase or (and) a lactosylceramide synthase.

**48.** The method according to claim 47, which further comprises inhibiting the activity of a GM3 synthase.

**49.** A method of preventing/treating arteriosclerosis, which comprises inhibiting the expression of a glucosylceramide synthase gene or (and) a lactosylceramide synthase gene.

**50.** The method according to claim 49, which further comprises inhibiting the expression of a GM3 synthase gene.

**51.** A method of preventing/treating arteriosclerosis, which comprises administering to a mammal an effective dose of a glucosylceramide synthase inhibitor or (and) a lactosylceramide synthase inhibitor.

**52.** The method according to claim 51, which further comprises administering an effective dose of a GM3 synthase inhibitor.

**53.** A method of preventing/treating arteriosclerosis, which comprises administering to a mammal an effective dose of an expression inhibitor of glucosylceramide synthase gene or (and) lactosylceramide synthase gene.

**54.** The method according to claim 53, which further comprises administering an effective dose of an expression inhibitor of GM3 synthase gene.

**55.** Use of a glucosylceramide synthase inhibitor or (and) a lactosylceramide synthase inhibitor to manufacture an agent for preventing/treating arteriosclerosis.

**56.** The use according to claim 55, which further comprises use of a GM3 synthase inhibitor.

**57.** Use of an expression inhibitor of glucosylceramide synthase gene or (and) lactosylceramide synthase gene to manufacture an agent for preventing/treating arteriosclerosis.

**58.** The use according to claim 57, which further comprises use of an expression inhibitor of GM3 synthase gene.

**INTERNATIONAL SEARCH REPORT**

International application No.
PCT/JP2005/016694

A. CLASSIFICATION OF SUBJECT MATTER
Int.Cl[7] A61K45/00, 38/57, 31/7088, 39/395, 45/06, 48/00, A61P3/06, 3/10, 9/10, 9/12, 13/12, 43/00, C12N15/09, C12Q1/527, 1/68, G01N33/15, 33/50//C12N9/88

According to International Patent Classification (IPC) or to both national classification and IPC

B. FIELDS SEARCHED

Minimum documentation searched (classification system followed by classification symbols)
Int.Cl[7] A61K45/00, 38/57, 31/7088, 39/395, 45/06, 48/00, A61P3/06, 3/10, 9/10, 9/12, 13/12, 43/00, C12N15/09, C12Q1/527, 1/68, G01N33/15, 33/50//C12N9/88

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
BIOSIS(STN), CAplus(STN), EMBASE(STN), MEDLINE(STN), SwissProt/PIR/Geneseq, JMEDPlus(JOIS)

C. DOCUMENTS CONSIDERED TO BE RELEVANT

| Category* | Citation of document, with indication, where appropriate, of the relevant passages | Relevant to claim No. |
|---|---|---|
| X<br>Y<br><br><br><br>A | WO 2002/55498 A1 (OXFORD GLYCOSCIENCES(UK) LTD.),<br>18 July, 2002 (18.07.02),<br>Abstract; Claims 10, 17<br>& EP 1362031 A1 & BR 200206433 A<br>& AU 2002219363 A1 & HU 200303891 A2<br>& US 2004/097551 A1 & JP 2004-517869 A<br>& ZA 200305118 A | 1,3-6,55<br>2,7-10, 13-22,25,26, 29,30,35-46, 56-58<br>11,12 |
| X<br>Y<br><br><br><br>A | CHATTERJEE, S., Sphingolipids in atherosclerosis and vascular biology, Arterioscler Thromb Vasc.Biol., 1998, Vol.18, No.10, p.1523-33, Abstract | 1,3-6,55<br>2,7-10, 13-22,25,26, 29,30,35-46, 56-58<br>11,12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>28 September, 2005 (28.09.05) | Date of mailing of the international search report<br>25 October, 2005 (25.10.05) |
| Name and mailing address of the ISA/<br>Japanese Patent Office<br><br>Facsimile No. | Authorized officer<br><br><br>Telephone No. |

Form PCT/ISA/210 (second sheet) (January 2004)

**INTERNATIONAL SEARCH REPORT**

International application No.
PCT/JP2005/016694

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br><br><br><br>A | CHATTERJEE, S. et al., Identification of a biologically active component in minimally oxidized low density lipoprotein (MM-LDL) responsible for aortic smooth muscle cell proliferation, Glycoconj J., 2004, Vol.20, No.5, p.331-8. Abstract | 1,3-6,55<br>2,7-10,<br>13-22,25,26,<br>29,30,35-46,<br>56-58<br>11,12 |
| X<br>Y<br><br><br><br>A | Williams, Nieshia Renee et al., The role of nicotine in the pathogenesis of atherosclerosis. FASEB Journal, 2002, Vol.16, No.5, pages A907 to A908. 678.8 | 1,3-6,55<br>2,7-10,<br>13-22,25,26,<br>29,30,35-46,<br>56-58<br>11,12 |
| X<br>Y<br><br><br><br>A | JP 11-318444 A (Kao Corp.),<br>24 November, 1999 (24.11.99),<br>Abstract; Claims; Par. No. [0017]<br>& JP 3545601 B2 | 1,3-6,55<br>2,7-10,<br>13-22,25,26,<br>29,30,35-46,<br>56-58<br>11,12 |
| X<br>Y | WO 2003/057874 A1 (Sumitomo Pharmaceuticals Co., Ltd.),<br>17 July, 2003 (17.07.03),<br>Abstract; Claims; sequence Nos. 1 to 3<br>& AU 2002367266 A1 & JP 2003-558172 A | 11,12<br>2,7,8,13-18,<br>39-41,45,46 |
| X<br>Y | WO 2001/36628 A1 (JOHN WAYNE CANCER INSTITUTE),<br>25 May, 2001 (25.05.01),<br>Abstract; Claims<br>& AU 200114533 A & US 2003/095953 A1 | 11,12<br>2,7,8,13-18,<br>39-41,45,46 |
| X<br>Y | WO 2001/68093 A1 (OXFORD GLYCOSCIENCES(UK) LTD.),<br>20 September, 2001 (20.09.01),<br>Claims<br>& AU 200140885 A & EP 1263436 A1<br>& US 2003/069200 A1 & JP 2003-534244 A | 11,12<br>2,7,8,13-18,<br>39-41,45,46 |
| X<br>Y | WO 2004/019900 A1 (SOCIETE DES PRODUITS NESTLE),<br>11 March, 2004 (11.03.04),<br>Claims<br>& AU 2003266313 A1 & EP 1539095 A1<br>& BR 200313808 A | 11,12<br>2,7,8,13-18,<br>39-41,45,46 |
| X<br>Y | DI, S.A. et al., Antisense to glucosylceramide synthase in human neuroepithelioma affects cell growth but not apoptosis, Cell Death Differ, 2002, Vol.9, No.6, p.693-5, Title | 11,12<br>2,7,8,13-18,<br>39-41,45,46 |

Form PCT/ISA/210 (continuation of second sheet) (January 2004)

**INTERNATIONAL SEARCH REPORT**

International application No.
PCT/JP2005/016694

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | DI, S.A. et al., Glucosylceramide synthase and its functional interaction with RTN-1C regulate chemotherapeutic-induced apoptosis in neuroepithelioma cells, Cancer Res., 2003, Vol.63, No.14, p.3860-5, Abstract | 11,12<br>2,7,8,13-18, 39-41,45,46 |
| Y | PANNU, R. et al., A novel role of lactosyl ceramide in the regulation of lipopoly saccharide/interferon-gamma-mediated inducible nitric oxide synthase gene expression: implications for neuroinflammatory diseases, J.Neurosci., 2004, Vol.24, No.26, p.5942-54, Abstract | 7-10,14,18, 20,22,24,26, 28,36,38,40, 42,44,46,48, 56,58 |
| Y | PROKAZOVA, N.V. et al., Sialylated lactosyl ceramides. Possible inducers of non-specific immunosuppression and atherosclerotic lesions, Eur.J.Biochem., 1988, Vol.172, No.1, pages 1 to 6, Abstract, page 4, left column, Par. No. [0004] | 7-10,14,18, 20,22,24,26, 28,36,38,40, 42,44,46,48, 56,58 |
| Y<br><br>A | GOLOVANOVA, N.K. et al., Development of antibody to human GM3 synthase and immunodetection of the enzyme in human tissues, Biochemistry(Mosc), March 2004, Vol.69, No.3, p.275-80, Abstract | 19-24,29, 35-46<br>3,4,11,12, 15,16,25,30, 35,37,39,41, 45 |
| Y | WO 2002/45740 A1 (CHILDRENS MEMORIAL HOSPITAL, US),<br>13 June, 2002 (13.06.02),<br>Claims; page 3, line 11 to page 4, line 10<br>& US 2002/122795 A1 & AU 200226060 A<br>& EP 1349567 A1 & GB 2388777 A<br>& JP 2004-531469 A | 19-22,29 |
| Y | CHATTERJEE, S., Lactosylceramide stimulates aortic smooth muscle cell proliferation, Biochem.Biophys.Res.Commun., 1991, Vol.181, No.2, p.554-61, Abstract | 8-10,14,18, 58 |
| A | JP 10-295371 A (Kao Corp.),<br>10 November, 1998 (10.11.98),<br>Abstract; sequence Nos. 5, 6<br>& JP 3578904 B2 | 5,6,11,12, 15,16,25,35, 37,39,41,43, 45 |
| A | US 2003/087396 A1 (KNOBBE MARTENS OLSON & BEAR LLP),<br>08 May, 2003 (08.05.03),<br>Abstract; sequences: 1, 2<br>& JP 2000-333682 A & US 6555371 B1 | 9,10,14,18, 26.36,38,40, 42,44,46 |

Form PCT/ISA/210 (continuation of second sheet) (January 2004)

**INTERNATIONAL SEARCH REPORT**

International application No. PCT/JP2005/016694

C (Continuation). DOCUMENTS CONSIDERED TO BE RELEVANT

| Category* | Citation of document, with indication, where appropriate, of the relevant passages | Relevant to claim No. |
|---|---|---|
| A | EP 890645 A2 (SEIKAGAKU KOGYO KABUSHIKI KAISHA), 13 January, 1999 (13.01.99), Abstract; sequences: 1, 2 & JP 11-018778 A | 9,10,14,18, 26.36,38,40, 42,44,46 |
| P,A | WO 2005/067971 A1 (Takeda Chemical Industries, Ltd.), 28 July, 2005 (28.07.05), Abstract; Claims; sequence Nos. 1, 2 (Family: none) | 7-10,14,18, 22,26,36,38, 42,44,46,56, 58 |

Form PCT/ISA/210 (continuation of second sheet) (January 2004)

**INTERNATIONAL SEARCH REPORT**

International application No.
PCT/JP2005/016694

**Box No. II Observations where certain claims were found unsearchable (Continuation of item 2 of first sheet)**

This international search report has not been established in respect of certain claims under Article 17(2)(a) for the following reasons:

1. [X] Claims Nos.: 23, 24, 27, 28, 31-34, 47-54
because they relate to subject matter not required to be searched by this Authority, namely:
Claims 23, 24, 27, 28, 31 to 34, and 47 to 54 pertain to methods for treatment of the human body by therapy and thus relate to a subject matter which this International Searching Authority is not required, under the provisions of the PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. [ ] As all required additional search fees were timely paid by the applicant, this international search report covers all searchable claims.

2. [X] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest.

[ ] No protest accompanied the payment of additional search fees.

Form PCT/ISA/210 (continuation of first sheet (2)) (January 2004)

INTERNATIONAL SEARCH REPORT

International application No.
PCT/JP2005/016694

Continuation of Box No.III of continuation of first sheet(2)

The technical feature common to claims 1-58 is a glucosylceramide synthase inhibitor or(and) lactosylceramide synthase inhibitor. However, as this technical feature even with respect to the use, relating to arteriosclerosis prevention/therapy, of glucosylceramide synthase inhibitor or(and) lactosylceramide synthase inhibitor is not novel, the special technical features of claims 1-58 concern the individual uses of individual glucosylceramide synthase inhibitors or(and) lactosylceramide synthase inhibitors. These inventions, as among the same there is no technical relationship involving one or more of the same or corresponding special technical features, cannot be recognized as being linked with each other so as to form a single general inventive concept. Accordingly, the following six inventions are involved.

1. Claims 1, 3-7 part thereof, 9 and 10 part thereof, 35-38, 43, 44, 47, 48, 51, 52, 55 and 56
   use, relating to arteriosclerosis prevention/therapy, of glucosylceramide synthase inhibitor or(and) lactosylceramide synthase inhibitor.

2. Claims 2, 3-7 part thereof, 8, 9 and 10 part thereof, 25-28, 30, 39-42, 45, 46, 49, 50, 53, 54, 57 and 58
   use, in arteriosclerosis prevention/therapy, of an expression inhibitor for glucosylceramide synthase gene or(and) an expression inhibitor for lactosylceramide synthase gene.

3. Claims 11-14
   an antisense polynucleotide containing a nucleotide sequence, or a part thereof, complementary to the polynucleotide sequence coding for protein having the amino acid sequence of SEQ ID NO: 1 or SEQ ID NO: 3, and use of the antisense polynucleotide.

4. Claims 15-18
   siRNA or shRNA against the polynucleotide coding for protein having the amino acid sequence of SEQ ID NO: 1 or SEQ ID NO: 3, and use of the siRNA or shRNA.

5. Claims 19-24 and 29
   use, relating to arteriosclerosis prevention/therapy, of an agent comprising an antibody against glucosylceramide synthase or(and) an antibody against lactosylceramide synthase.

6. Claims 31-34
   a method of diagnosing arteriosclerosis, characterized in that the level of glucosylceramide or(and) lactosylceramide is measured.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0378624 A


### Non-patent literature cited in the description


- **FUSTER, V. et al.** *N. Engl. J. Med.,* 1992, vol. 326, 242-250
- **EHARA, S. et al.** *Circulation,* 2001, vol. 103, 1955-1960
- *Biochim. Biophys. Acta,* 2001, vol. 1525, 1-12
- *Wakaru-Jikken-Igaku Series: Glycobiology,* 2002, 44-52
- *Biochem. Biophys. Res. Commun.,* 1991, vol. 181, 554-61
- *J. Biol. Chem.,* 1998, vol. 273, 34349
- *J. Biol. Chem.,* 2002, vol. 277, 3085-3092
- *Methods in ENZYMOLOGY,* 2000, vol. 311, 50-59
- *Methods in ENZYMOLOGY,* 2000, vol. 311, 73-81
- Kiso-Seikagaku-Jikkenho. 2000, vol. 5, 135-141
- *Methods in ENZYMOLOGY,* 2000, vol. 311, 82-94
- *J. Biol. Chem.,* 2002, vol. 277, 47028-47034
- *J. Cell. Phys.,* 1989, vol. 141, 573-583
- **M. BODANSZKY ; M.A. ONDETTI.** Peptide Synthesis. Interscience Publishers, 1966
- **SCHROEDER ; LUEBKE.** The Peptide. Academic Press, 1965
- **NOBUO IZUMIYA et al.** Peptide Gosei-no-Kiso to Jikken. Maruzen Co, 1975
- **HARUAKI YAJIMA ; SHUNPEI SAKAKIBARA.** Seikagaku Jikken Koza. *Tanpakushitsu no Kagaku,* 1977, vol. IV, 205
- Zoku Iyakuhin no Kaihatsu. Peptide Synthesis. Hirokawa Shoten, vol. 14
- **J. SAMBROOK et al.** Molecular Cloning. Cold Spring Harbor Lab. Press, 1989
- *Proc. Natl. Acad. Sci. U.S.A.,* 1968, vol. 60, 160
- *Nucleic Acids Research,* 1981, vol. 9, 309
- *Journal of Molecular Biology,* 1978, vol. 120, 517
- *Journal of Molecular Biology,* 1969, vol. 41, 459
- *Genetics,* 1954, vol. 39, 440
- *Gene,* 1983, vol. 24, 255
- *Journal of Biochemistry,* 1984, vol. 95, 87
- **VAUGHN, J. L. et al.** *In Vivo,* 1977, vol. 13, 213-217
- **MAEDA et al.** *Nature,* 1985, vol. 315, 592
- *Proc. Natl. Acad. Sci. U.S.A.,* 1972, vol. 69, 2110
- *Gene,* 1982, vol. 17, 107
- *Molecular & General Genetics,* 1979, vol. 168, 111
- *Methods in Enzymology,* 1991, vol. 194, 182-187
- *Proc. Natl. Acad. Sci. U.S.A.,* 1978, vol. 75, 1929
- *Bio/Technology,* 1988, vol. 6, 47-55
- Shin Saibo Kogaku Jikken Protocol. Saibo Kogaku. Shujunsha, 1995, 263-267
- *Virology,* 1973, vol. 52, 456
- **MILLER.** Journal of Experiments in Molecular Genetics. Cold Spring Harbor Laboratory, 1972, 431-433
- **BOSTIAN, K. L. et al.** *Proc. Natl. Acad. Sci. U.S.A.,* 1980, vol. 77, 4505
- **BITTER, G. A. et al.** *Proc. Natl. Acad. Sci. U.S.A.,* 1984, vol. 81, 5330
- **GRACE, T. C. C.** *Nature,* 1962, vol. 195, 788
- *Science,* 1952, vol. 122, 501
- *Virology,* 1959, vol. 8, 396
- *The Journal of the American Medical Association,* 1967, vol. 199, 519
- *Proceeding of the Society for the Biological Medicine,* 1950, vol. 73, 1
- **KOEHLER ; MILSTEIN.** *Nature,* 1975, vol. 256, 495
- **J. KAWAKAMI et al.** *Pharm. Tech. Japan,* 1992, vol. 8, 247
- *PHARM. TECH. JAPAN,* 1992, vol. 8, 395
- Antisense Research and Applications. CRC Press, 1993
- *J. Biol. Chem.,* 1999, vol. 274, 19707-19713
- *J. Biol. Chem.,* 2001, vol. 276, 43564-43569
- *Pro. Natl. Acad. Sci.,* 1991, vol. 88, 4931-4935
- Radioimmunoassay. Kodansha, 1974
- Sequel to the Radioimmunoassay. Kodansha, 1979

- Enzyme immunoassay. Igakushoin, 1978
- Immunoenzyme assay. Igakushoin, 1982
- Immunoenzyme assay. Igakushoin, 1987
- Immunochemical Techniques (Part A. Methods in ENZYMOLOGY. vol. 70
- Immunochemical Techniques (Part B. METHODS IN ENZYMOLOGY. vol. 73
- Immunochemical Techniques (Part C. METHODS IN ENZYMOLOGY. vol. 74
- Immunochemical Techniques (Part D: Selected Immunoassays. METHODS IN ENZYMOLOGY. vol. 84
- Immunochemical Techniques (Part E: Monoclonal Antibodies and General Immunoassay Methods. MEHODS IN ENZYMOLOGY. vol. 92
- Immunochemical Techniques (Part I: Hybridoma Technology and Monoclonal Antibodies. METHODS IN ENZYMOLOGY. Academic Press Publishing, vol. 121
- *Genomics,* 1989, vol. 5, 874-879
- *Proceedings of the National Academy of Sciences of the United States of America,* 1989, vol. 86, 2766-2770
- *Nature,* 2001, vol. 411, 494
- *TRENDS in Molecular Medicine,* 2001, vol. 7, 221
- *Annual Review of Medicine,* 2005, vol. 56, 555-583
- **M. J. EVANS ; M. H. KAUFMAN.** *Nature,* 1981, vol. 292, 154
- **G. R. MARTIN.** *Proc. Natl. Acad. Sci. U.S.A.,* 1981, vol. 78, 7634
- **T. C. DOETSCHMAN et al.** *Journal of Embryology Experimental Morphology,* 1985, vol. 87, 27
- *Glycoconjugate J.,* 1996, vol. 13, 481-486
- *J. Biochem. (Tokyo,* 1995, vol. 117, 766-773
- *Int. J. Cancer.,* 1980, vol. 26, 171-176
- *J. Gen. Virol.,* 1977, vol. 36, 59-74